# EUROPEAN PATENT SPECIFICATION

(11) **EP 3 125 962 B1**
(45) Date of publication and mention of the grant of the patent: **21.08.2019**
(21) Application number: 15717728.8
(22) Date of filing: 01.04.2015
(51) Int. Cl.: A61L 27/36

(54) **MICROBIAL ENZYMES FOR REDUCTION OF ALPHA-GALACTOSE FROM COLLAGEN BASED TISSUE**
MIKROBIELLE ENZYME ZUR REDUKTION VON ALPHA-GALACTOSE AUS COLLAGENHALTIGEN GEWEBE
ENZYMES MICROBIENNES POUR LA RÉDUCTION D'ALPHA-GALACTOSE DANS DES TISSUS À BASE DE COLLAGÈNE

(30) Priority: 01.04.2014 US 201461973470 P
(43) Date of publication of application: 08.02.2017
(73) Proprietor: LifeCell Corporation, Madison, New Jersey 07940 (US)
(72) Inventor: OWENS, Rick, T., Stewartsville, NJ 08886 (US); GEORGE, Niraj, P.E., Havertown, PA 19083 (US)
(74) Representative: Ashton, Timothy
(86) International application number: PCT/US2015/023855
(87) International publication number: WO 2015/153745

(56) References cited:
- WO-A1-99/12555
- US-B2- 7 951 552

### SEQUENCE LISTING

<110> LIFECELL CORPORATION
<120> MICROBIAL ENZYMES FOR REDUCTION OF ALPHA-GALACTOSE FROM COLLAGEN BASED TISSUE
<130> LIF.1164PCT
<140>
   <141>
<150> 61/973,470
   <151> 2014-04-01
<160> 8
<170> PatentIn version 3.5
<210> 1
   <211> 1677
   <212> DNA
   <213> Trichoderma reesei
<400> 1
   gacaagcgaa ggccttactg caagaacgag tggcagtcta catcttctac agcggacgga      60
   gagattatcg aactgcttgt atcatagctg gaacacacaa tgacccctca ctcgattgac     120
   cgggccgcga ggccttccgt ctggagtggc cttgctcttc tcctctctac agcacacgca     180
   atcgtgatgc ctgatggagt gactggaaaa gttccaagtc tggggtggaa ctcgtggaat     240
   gcctaccact gcgatatcga tgaaagcaag tttctctcgg ccgccgaagt cattgtgagc     300
   tctgggcttc tcgacgcagg ctacaattat gtcaatattg atgactgctg gtcgatgaaa     360
   gacggtcgtg tggacggcca tattgcagtc aacacaactc gcttccctga cggcattgat     420
   gggctggcga agaaggtcca cgacttgggc ttgaagttgg gcatttacag cactgctggg     480
   actgcaactt gtgctggcta ccctgccagt cttggctacg aggacgtcga tgccgctgat     540
   tttgccgact ggggcgtgga ctatctgaaa tatgacaatt gcaacgtccc ttcagactgg     600
   caagatgaat acgtcgcctg cgcccccgac gccgtccaaa ccggccccaa cggcacctgc     660
   tcaaccgccc ttgagccaaa cctcgcccct ccgggctacg actggagcac ttccaagtca     720
   gccgagcgct tcaacgccat gaggaacgcc ctggcgaagc agagccgcga gatcgtgctc     780
   agcctgtgca tctggggagt ggccgacgtc ttctcctggg gcaacgagac aggcatcagc     840
   tggcgcatga gcggcgatat ttcgcccgaa tggggctccg tgacgcacat catcaacatg     900
   aattcgttca agatgaattc cgtcggcttc tggggccaca acgacgcgga tatactcgag     960
   gtcggcaacg gcaacctgac ggctgctgag acgcggacgc actttgcgct gtgggcggcc    1020
   atgaagtcgc cgctgctgat cgggacggat cttgctcagt tgtcgcagga gaacattgag    1080
   ttgctgaaga ataagcatct gctggcgttt aaccaggaca gcgtctacgg tcagcctgcc    1140
   acgccctata aatggggcgt caaccctgac tggaccttta actatacgaa ccctgccgag     1200
   tactgggccg gtccatcgtc aaaggggcat ctggtgctga tgatgaacac gctggatcac     1260
   acggtgagaa aggaggccaa gtggtctgag attccggggc tgtctgcggg acggtatgag     1320
   gtccgggatg tgtggacgga caagagcctt gggtgcctca gctcgtacaa gacggctgtt     1380
   gcagctcatg acaccgctgt tattctggtt ggcaagaagt gccgaaactg gtgatggggt     1440
   cggaagagga tggatatcga ctggacctct ggttgaatgc ttgtcatcgt ctgcatcctg     1500
   acgggagccg gaaggactgt ggctgctgtc tgcttcagat ccagatggaa gcacccttat     1560
   ccagactatc cagaaatcac aagccaattg caggattcca gtcttctcag ttactaggag     1620
   gtgaataagt aaggtagaga tggctgactt gtcctgctaa aaaaaaaaaa aaaaaaa        1677
<210> 2
   <211> 444
   <212> PRT
   <213> Trichoderma reesei
<400> 2
   Met Thr Pro His Ser Ile Asp Arg Ala Ala Arg Pro Ser Val Trp Ser
   1               5                   10                  15
   Gly Leu Ala Leu Leu Leu Ser Thr Ala His Ala Ile Val Met Pro Asp
               20                  25                  30
   Gly Val Thr Gly Lys Val Pro Ser Leu Gly Trp Asn Ser Trp Asn Ala
           35                  40                  45
   Tyr His Cys Asp Ile Asp Glu Ser Lys Phe Leu Ser Ala Ala Glu Val
       50                  55                  60
   Ile Val Ser Ser Gly Leu Leu Asp Ala Gly Tyr Asn Tyr Val Asn Ile
   65                  70                  75                  80
   Asp Asp Cys Trp Ser Met Lys Asp Gly Arg Val Asp Gly His Ile Ala
                   85                  90                  95
   Val Asn Thr Thr Arg Phe Pro Asp Gly Ile Asp Gly Leu Ala Lys Lys
               100                 105                 110
   Val His Asp Leu Gly Leu Lys Leu Gly Ile Tyr Ser Thr Ala Gly Thr
           115                 120                 125
   Ala Thr Cys Ala Gly Tyr Pro Ala Ser Leu Gly Tyr Glu Asp Val Asp
       130                 135                 140
   Ala Ala Asp Phe Ala Asp Trp Gly Val Asp Tyr Leu Lys Tyr Asp Asn
   145                 150                 155                 160
   Cys Asn Val Pro Ser Asp Trp Gln Asp Glu Tyr Val Ala Cys Ala Pro
                   165                 170                 175
   Asp Ala Val Gln Thr Gly Pro Asn Gly Thr Cys Ser Thr Ala Leu Glu
               180                 185                 190
   Pro Asn Leu Ala Pro Pro Gly Tyr Asp Trp Ser Thr Ser Lys Ser Ala
           195                 200                 205
   Glu Arg Phe Asn Ala Met Arg Asn Ala Leu Ala Lys Gln Ser Arg Glu
       210                 215                 220
   Ile Val Leu Ser Leu Cys Ile Trp Gly Val Ala Asp Val Phe Ser Trp
   225                 230                 235                 240
   Gly Asn Glu Thr Gly Ile Ser Trp Arg Met Ser Gly Asp Ile Ser Pro
                   245                 250                 255
   Glu Trp Gly Ser Val Thr His Ile Ile Asn Met Asn Ser Phe Lys Met
               260                 265                 270
   Asn Ser Val Gly Phe Trp Gly His Asn Asp Ala Asp Ile Leu Glu Val
           275                 280                 285
   Gly Asn Gly Asn Leu Thr Ala Ala Glu Thr Arg Thr His Phe Ala Leu
       290                 295                 300
   Trp Ala Ala Met Lys Ser Pro Leu Leu Ile Gly Thr Asp Leu Ala Gln
   305                 310                 315                 320
   Leu Ser Gln Glu Asn Ile Glu Leu Leu Lys Asn Lys His Leu Leu Ala
                   325                 330                 335
   Phe Asn Gln Asp Ser Val Tyr Gly Gln Pro Ala Thr Pro Tyr Lys Trp
               340                 345                 350
   Gly Val Asn Pro Asp Trp Thr Phe Asn Tyr Thr Asn Pro Ala Glu Tyr
           355                 360                 365
   Trp Ala Gly Pro Ser Ser Lys Gly His Leu Val Leu Met Met Asn Thr
       370                 375                 380
   Leu Asp His Thr Val Arg Lys Glu Ala Lys Trp Ser Glu Ile Pro Gly
   385                 390                 395                 400
   Leu Ser Ala Gly Arg Tyr Glu Val Arg Asp Val Trp Thr Asp Lys Ser
                   405                 410                 415
   Leu Gly Cys Leu Ser Ser Tyr Lys Thr Ala Val Ala Ala His Asp Thr
               420                 425                 430
   Ala Val Ile Leu Val Gly Lys Lys Cys Arg Asn Trp
           435                 440
<210> 3
   <211> 2438
   <212> DNA
   <213> Trichoderma reesei
<400> 3
   cacagttggc cccggactct gtggtggcca atcttcacga tgctcggcgc tccctctcct 60
   agaaggctgg cggacgtcct cgccgtgacc gcgggactgg tggcctctgt tagggcagca 120
   agtcccatct ccgtgtctgg caagtcgttt gccctcaacg gcgacaacgt ctcgtaccgc 180
   ttccacgtcg acgacgactc taaggacctc atcggcgacc actttggcgg ccctgccacg 240
   gaagatggcg tcttcccccc catcatcggc cccatccagg gctgggtcga cctcatcggc 300
   cggcagcggc gcgagttccc cgacctgggc cgcggcgact ttcgcacgcc cgcggtgcac 360
   atccggcagg cggcgggcta cacggtcagc gacttccagt acaagtcgca ccgcgtcgtc 420
   gagggcaagc cggcgctgcg cggcctgccg tcgacgtttg gcgacgccgg cgatgtgtcg 480
   actctggtcg tgcacatgta tgataactac agctccgtgg ccgccgacct gacctactcc 540
   atcttcccca aatacgacgc cattgtgcgc agcgtcaaca tcaccaacat gggcaagggt 600
   aacatcacca ttgagaagct cgccagcttg agcgtcgatc tgccgtatga ggactttgac 660
   atgctggagc tcaagggtga ctgggctcgc gagggaaagc ggctgcgtcg caaggttgac 720
   tacggctctc agggtttcgg gagcacgact ggctattctt cccatctcca caaccccttc 780
   ttctcgctca tcacgcctac gacgaccgag tcccaaggag aggcatgggg cttctccctt 840
   gtgtacactg gctccttctc cgtcgaggtc gaaaagggtt cgcagggtct cacgcgagcc 900
   gccattggcg tcaaccccta tcaactgtcg tggccgttgg gccctggcga gaccttcagc 960
   agccccgagg cggttgccgt cttctctacc actggcgttg gcggaatgtc gcgaaagttc 1020
   cacaacctgt accgcaagca tctgatcaag agcaaattcg cgacgcagat gcaccccgtc 1080
   ttgctcaaca gctgggaggg cctcggcttc gactacaacg acaccaccat tctgcatctg 1140
   gcgcaggagt ctgccgatct cggcatcaag ctgtttgtgc tggatgatgg ctggtttggc 1200
   gtcaagcatc ctcgagttag tgacaatgct ggcctgggcg actgggaggc gaaccccaag 1260
   aggttcccgc agggcctgcc agacttcatt agcgacgtga caaagctcaa ggtggccaac 1320
   tcctctgatc atctgcagtt tggcctctgg ttcgagcctg aaatggtcaa ccccaactca 1380
   accctataca tggaacaccc ggactgggcg attcacgccg ggtcgtaccc tcgtaccttg 1440
   acgaggaacc agctggtgct caacgtcgct ctcccagagg tgcaggattt catcattgag 1500
   tcgctgtcca acattctgag caacgccagc atttcgtacg tcaagtggga caacaaccgc 1560
   ggcatccacg aggcccctta ccccgggctc gactacgcct acatgctggg cctgtaccgc 1620
   gtctttgaca cgctgtcgtc aaagttcccc aatgttcgct gggagggttg cgcgtctggc 1680
   ggcggccgct tcgatcccgg cgtgctgcag tactttcctc acatctggac gtctgacgac 1740
   acggatgccg tggagcgcat tgcgatccag tttggcacgt cgctcgtgta tccgccgtcg 1800
   gccatgggag cccacgtctc tgccgtaccg aacggccaga cgcagcgcac gacgtcgatt 1860
   gccttccgcg cccacgttgc catgatggga ggttcgtttg gcttcgagct cacccctgcg 1920
   gagatgccgg aggacgacaa ggcgcagatc ccgggcatca ttgcgctggc ggaaaaggtg 1980
   aaccccattg ttgtcaaggg cgacatgtgg cggctgagcc tgccggagga gtccaactgg 2040
   ccggcggcgc tcttcatctc gcaggacggc agccaggcgg tgctgttcta cttccagatc 2100
   cgggccaaca tcaacaacgc gtggccggtg ctgcggctgc agggcctgga tgcgtcggcc 2160
   aagtacaaga ttgacggcaa ccagacgttc tcgggggcga cgctgatgaa catcgggctg 2220
   cagtatcagt tcaatgggga ctatgacagc aaggtggtgt ttttggagaa gcagacatga 2280
   gctgattttg tgaacccatc accatgacct agatgaccta caggctttgt ttgatgcatc 2340
   gtttcttcag tagcacttat gtatggtagc ttagcattca gtagttaata cacagcaggc 2400
   gttttctgac tgaaaaaaga aaaaaaaaaa aaaaaaaa 2438
<210> 4
   <211> 746
   <212> PRT
   <213> Trichoderma reesei
<400> 4
   Met Leu Gly Ala Pro Ser Pro Arg Arg Leu Ala Asp Val Leu Ala Val
   1               5                   10                  15
   Thr Ala Gly Leu Val Ala Ser Val Arg Ala Ala Ser Pro Ile Ser Val
               20                  25                  30
   Ser Gly Lys Ser Phe Ala Leu Asn Gly Asp Asn Val Ser Tyr Arg Phe
           35                  40                  45
   His Val Asp Asp Asp Ser Lys Asp Leu Ile Gly Asp His Phe Gly Gly
       50                  55                  60
   Pro Ala Thr Glu Asp Gly Val Phe Pro Pro Ile Ile Gly Pro Ile Gln
   65                  70                  75                  80
   Gly Trp Val Asp Leu Ile Gly Arg Gln Arg Arg Glu Phe Pro Asp Leu
                   85                  90                  95
   Gly Arg Gly Asp Phe Arg Thr Pro Ala Val His Ile Arg Gln Ala Ala
               100                 105                 110
   Gly Tyr Thr Val Ser Asp Phe Gln Tyr Lys Ser His Arg Val Val Glu
           115                 120                 125
   Gly Lys Pro Ala Leu Arg Gly Leu Pro Ser Thr Phe Gly Asp Ala Gly
       130                 135                 140
   Asp Val Ser Thr Leu Val Val His Met Tyr Asp Asn Tyr Ser Ser Val
   145                 150                 155                 160
   Ala Ala Asp Leu Thr Tyr Ser Ile Phe Pro Lys Tyr Asp Ala Ile Val
                   165                 170                 175
   Arg Ser Val Asn Ile Thr Asn Met Gly Lys Gly Asn Ile Thr Ile Glu
               180                 185                 190
   Lys Leu Ala Ser Leu Ser Val Asp Leu Pro Tyr Glu Asp Phe Asp Met
           195                 200                 205
   Leu Glu Leu Lys Gly Asp Trp Ala Arg Glu Gly Lys Arg Leu Arg Arg
       210                 215                 220
   Lys Val Asp Tyr Gly Ser Gln Gly Phe Gly Ser Thr Thr Gly Tyr Ser
   225                 230                 235                 240
   Ser His Leu His Asn Pro Phe Phe Ser Leu Ile Thr Pro Thr Thr Thr
                   245                 250                 255
   Glu Ser Gln Gly Glu Ala Trp Gly Phe Ser Leu Val Tyr Thr Gly Ser
               260                 265                 270
   Phe Ser Val Glu Val Glu Lys Gly Ser Gln Gly Leu Thr Arg Ala Ala
           275                 280                 285
   Ile Gly Val Asn Pro Tyr Gln Leu Ser Trp Pro Leu Gly Pro Gly Glu
       290                 295                 300
   Thr Phe Ser Ser Pro Glu Ala Val Ala Val Phe Ser Thr Thr Gly Val
   305                 310                 315                 320
   Gly Gly Met Ser Arg Lys Phe His Asn Leu Tyr Arg Lys His Leu Ile
                   325                 330                 335
   Lys Ser Lys Phe Ala Thr Gln Met His Pro Val Leu Leu Asn Ser Trp
               340                 345                 350
   Glu Gly Leu Gly Phe Asp Tyr Asn Asp Thr Thr Ile Leu His Leu Ala
           355                 360                 365
   Gln Glu Ser Ala Asp Leu Gly Ile Lys Leu Phe Val Leu Asp Asp Gly
       370                 375                 380
   Trp Phe Gly Val Lys His Pro Arg Val Ser Asp Asn Ala Gly Leu Gly
   385                 390                 395                 400
   Asp Trp Glu Ala Asn Pro Lys Arg Phe Pro Gln Gly Leu Pro Asp Phe
                   405                 410                 415
   Ile Ser Asp Val Thr Lys Leu Lys Val Ala Asn Ser Ser Asp His Leu
               420                 425                 430
   Gln Phe Gly Leu Trp Phe Glu Pro Glu Met Val Asn Pro Asn Ser Thr
           435                 440                 445
   Leu Tyr Met Glu His Pro Asp Trp Ala Ile His Ala Gly Ser Tyr Pro
       450                 455                 460
   Arg Thr Leu Thr Arg Asn Gln Leu Val Leu Asn Val Ala Leu Pro Glu
   465                 470                 475                 480
   Val Gln Asp Phe Ile Ile Glu Ser Leu Ser Asn Ile Leu Ser Asn Ala
                   485                 490                 495
   Ser Ile Ser Tyr Val Lys Trp Asp Asn Asn Arg Gly Ile His Glu Ala
               500                 505                 510
   Pro Tyr Pro Gly Leu Asp Tyr Ala Tyr Met Leu Gly Leu Tyr Arg Val
           515                 520                 525
   Phe Asp Thr Leu Ser Ser Lys Phe Pro Asn Val Arg Trp Glu Gly Cys
       530                 535                 540
   Ala Ser Gly Gly Gly Arg Phe Asp Pro Gly Val Leu Gln Tyr Phe Pro
   545                 550                 555                 560
   His Ile Trp Thr Ser Asp Asp Thr Asp Ala Val Glu Arg Ile Ala Ile
                   565                 570                 575
   Gln Phe Gly Thr Ser Leu Val Tyr Pro Pro Ser Ala Met Gly Ala His
               580                 585                 590
   Val Ser Ala Val Pro Asn Gly Gln Thr Gln Arg Thr Thr Ser Ile Ala
           595                 600                 605
   Phe Arg Ala His Val Ala Met Met Gly Gly Ser Phe Gly Phe Glu Leu
       610                 615                 620
   Thr Pro Ala Glu Met Pro Glu Asp Asp Lys Ala Gln Ile Pro Gly Ile
   625                 630                 635                 640
   Ile Ala Leu Ala Glu Lys Val Asn Pro Ile Val Val Lys Gly Asp Met
                   645                 650                 655
   Trp Arg Leu Ser Leu Pro Glu Glu Ser Asn Trp Pro Ala Ala Leu Phe
               660                 665                 670
   Ile Ser Gln Asp Gly Ser Gln Ala Val Leu Phe Tyr Phe Gln Ile Arg
           675                 680                 685
   Ala Asn Ile Asn Asn Ala Trp Pro Val Leu Arg Leu Gln Gly Leu Asp
       690                 695                 700
   Ala Ser Ala Lys Tyr Lys Ile Asp Gly Asn Gln Thr Phe Ser Gly Ala
   705                 710                 715                 720
   Thr Leu Met Asn Ile Gly Leu Gln Tyr Gln Phe Asn Gly Asp Tyr Asp
                   725                 730                 735
   Ser Lys Val Val Phe Leu Glu Lys Gln Thr
               740                 745
<210> 5
   <211> 2004
   <212> DNA
   <213> Trichoderma reesei
<400> 5
   gacaagccaa aggcgcaaag cgttcagtta catcgcagca caatgtcgcc cagtgctgca 60
   gttctcattc ccctcgcagc ggcagttctg cttcgtcctg tggtcggtca aacgcaatgc 120
   ggcggcaatc tgtacactcc ggggacgctc aacttcactc tggagtgcta caatgcgttt 180
   caggactgcg tcgctcagtt tgaggccaac gcaagccaag tcgactgcaa cgacggcaag 240
   ggaaacctgt tcatgcaaca acaggccaac ttgggggcct cgccaggaag ccagaacaac 300
   gacgccatca ttgcctttca ggacattcgc gatctctgtc tgctcagcgg ttcaacaact 360
   gcaacgtggg gatatagcga caaccagtgg tattgggcgg ctgccgaaga tgcctgctac 420
   acaaacgatc ccacgaggac cgacgttgtc aagactcacc cggcgccgtt ttgcatccag 480
   aaccgcgact cttcactgcc tgagtgctac ccacagccgg atgccacccc tcccggcggc 540
   ccactcaagg tcatcaagac ggccaagacg cgaaacgggt tcaagtcctc agcccgaggc 600
   tggaatacct acggcgtcca agctctggtc aacggttccc aggtcgtgcc gtcctttgct 660
   ggacagtcgg gtctgttcta cacccagaag ttcgtcgaga ctcagtgtgg agttcttgcc 720
   cgacccgagt tcaagaaggc tgggtacgat ctctgcagcc ttgattcggg ttggcaggct 780
   actaccgccg ttgatcagca tggtcgaatc atctacaaca ccacgcgatt caacctcccc 840
   gagcttgctt catggctaca caagagggat ttgaagctcg gcgtatacat tacccctggc 900
   gtgccatgtc tggctcacaa ccaaaccatc ctcggcacca acatcaagat caaggatgtc 960
   ttgaatggga acaacgatca gatcaactgt gactttgact tccgcaaaga tggtgtccag 1020
   cagtggcacg attccgtcgt cgcacaatgg gcttcctggg gcgtggacat gctcaaactg 1080
   gactttctga cgcctggctc cccttccaat ggcgcaaacc tcgcgtgtga cagttcagat 1140
   gctgttcgag cataccagaa ggcaatcaag aagtcaggac gaaaaattcg cctcgacatc 1200
   tcgtggaagc tttgccgcaa cgaaacctgg ctacctatct ggagcgacct tgctgagtca 1260
   atgcgcactg atcaggatct cgacaactac ggcaccaaca ctttgatggc atggcaggtc 1320
   ggccagcgcg cgattgagaa ctacaggcag tacatcggtt tgcaagcgca gagaaatgtc 1380
   cccctcacga tctatcctga tatggatgct cttttcacgg tcaaccccga gcatctcgcc 1440
   ggtgtaaacg acactattcg ctatacggtt cagaaccact ggcttggagc tggcgccaac 1500
   ctcatcattg gtggcgatat ggagcaggtc gatgctctgg ggctcaagct gactaccagc 1560
   aagcaatcga ttgatgcggc agactttttt gcaaagtatc ccatgcagcc tcgtaacccc 1620
   ggaaccggaa gcaacgccgc caagcagctc caggcctgga tcggtggccc ttcggatgac 1680
   cacgaggctt atgtgctcat tgtcaactac gggccagact tggggaatgg tggcttttca 1740
   accaagctgt atggaaagca gaaagtgaca gtgtcgttga aggatcttgg tatctctggc 1800
   tccgcctgga cttttaccga catatggtcc ggcaagtcca gcagagtgac tgggtcctac 1860
   tctgcctggc tcaccgaggg cgagtcccag cttctgcgcc tgaaaaggac tcactagtga 1920
   acacgaagac gtatagaggt ttctagagtt tagcgaagca atacacttta taatccctaa 1980
   aaaaaaaaaa aaaaaaaaaa aaaa 2004
<210> 6
   <211> 624
   <212> PRT
   <213> Trichoderma reesei
<400> 6
   Met Ser Pro Ser Ala Ala Val Leu Ile Pro Leu Ala Ala Ala Val Leu
   1 5 10 15
   Leu Arg Pro Val Val Gly Gln Thr Gln Cys Gly Gly Asn Leu Tyr Thr
               20                  25                  30
   Pro Gly Thr Leu Asn Phe Thr Leu Glu Cys Tyr Asn Ala Phe Gln Asp
           35                  40                  45
   Cys Val Ala Gln Phe Glu Ala Asn Ala Ser Gln Val Asp Cys Asn Asp
       50                  55                  60
   Gly Lys Gly Asn Leu Phe Met Gln Gln Gln Ala Asn Leu Gly Ala Ser
   65                  70                  75                  80
   Pro Gly Ser Gln Asn Asn Asp Ala Ile Ile Ala Phe Gln Asp Ile Arg
                   85                  90                  95
   Asp Leu Cys Leu Leu Ser Gly Ser Thr Thr Ala Thr Trp Gly Tyr Ser
               100                 105                 110
   Asp Asn Gln Trp Tyr Trp Ala Ala Ala Glu Asp Ala Cys Tyr Thr Asn
           115                 120                 125
   Asp Pro Thr Arg Thr Asp Val Val Lys Thr His Pro Ala Pro Phe Cys
       130                 135                 140
   Ile Gln Asn Arg Asp Ser Ser Leu Pro Glu Cys Tyr Pro Gln Pro Asp
   145                 150                 155                 160
   Ala Thr Pro Pro Gly Gly Pro Leu Lys Val Ile Lys Thr Ala Lys Thr
                   165                 170                 175
   Arg Asn Gly Phe Lys Ser Ser Ala Arg Gly Trp Asn Thr Tyr Gly Val
               180                 185                 190
   Gln Ala Leu Val Asn Gly Ser Gln Val Val Pro Ser Phe Ala Gly Gln
           195                 200                 205
   Ser Gly Leu Phe Tyr Thr Gln Lys Phe Val Glu Thr Gln Cys Gly Val
       210                 215                 220
   Leu Ala Arg Pro Glu Phe Lys Lys Ala Gly Tyr Asp Leu Cys Ser Leu
   225                 230                 235                 240
   Asp Ser Gly Trp Gln Ala Thr Thr Ala Val Asp Gln His Gly Arg Ile
                   245                 250                 255
   Ile Tyr Asn Thr Thr Arg Phe Asn Leu Pro Glu Leu Ala Ser Trp Leu
               260                 265                 270
   His Lys Arg Asp Leu Lys Leu Gly Val Tyr Ile Thr Pro Gly Val Pro
           275                 280                 285
   Cys Leu Ala His Asn Gln Thr Ile Leu Gly Thr Asn Ile Lys Ile Lys
       290                 295                 300
   Asp Val Leu Asn Gly Asn Asn Asp Gln Ile Asn Cys Asp Phe Asp Phe
   305                 310                 315                 320
   Arg Lys Asp Gly Val Gln Gln Trp His Asp Ser Val Val Ala Gln Trp
                   325                 330                 335
   Ala Ser Trp Gly Val Asp Met Leu Lys Leu Asp Phe Leu Thr Pro Gly
               340                 345                 350
   Ser Pro Ser Asn Gly Ala Asn Leu Ala Cys Asp Ser Ser Asp Ala Val
           355                 360                 365
   Arg Ala Tyr Gln Lys Ala Ile Lys Lys Ser Gly Arg Lys Ile Arg Leu
       370                 375                 380
   Asp Ile Ser Trp Lys Leu Cys Arg Asn Glu Thr Trp Leu Pro Ile Trp
   385                 390                 395                 400
   Ser Asp Leu Ala Glu Ser Met Arg Thr Asp Gln Asp Leu Asp Asn Tyr
                   405                 410                 415
   Gly Thr Asn Thr Leu Met Ala Trp Gln Val Gly Gln Arg Ala Ile Glu
               420                 425                 430
   Asn Tyr Arg Gln Tyr Ile Gly Leu Gln Ala Gln Arg Asn Val Pro Leu
           435                 440                 445
   Thr Ile Tyr Pro Asp Met Asp Ala Leu Phe Thr Val Asn Pro Glu His
       450                 455                 460
   Leu Ala Gly Val Asn Asp Thr Ile Arg Tyr Thr Val Gln Asn His Trp
   465                 470                 475                 480
   Leu Gly Ala Gly Ala Asn Leu Ile Ile Gly Gly Asp Met Glu Gln Val
                   485                 490                 495
   Asp Ala Leu Gly Leu Lys Leu Thr Thr Ser Lys Gln Ser Ile Asp Ala
               500                 505                 510
   Ala Asp Phe Phe Ala Lys Tyr Pro Met Gln Pro Arg Asn Pro Gly Thr
               515                 520                 525
   Gly Ser Asn Ala Ala Lys Gln Leu Gln Ala Trp Ile Gly Gly Pro Ser
       530                 535                 540
   Asp Asp His Glu Ala Tyr Val Leu Ile Val Asn Tyr Gly Pro Asp Leu
   545                 550                 555                 560
   Gly Asn Gly Gly Phe Ser Thr Lys Leu Tyr Gly Lys Gln Lys Val Thr
                   565                 570                 575
   Val Ser Leu Lys Asp Leu Gly Ile Ser Gly Ser Ala Trp Thr Phe Thr
               580                 585                 590
   Asp Ile Trp Ser Gly Lys Ser Ser Arg Val Thr Gly Ser Tyr Ser Ala
           595                 600                 605
   Trp Leu Thr Glu Gly Glu Ser Gln Leu Leu Arg Leu Lys Arg Thr His
       610                 615                 620
<210> 7
   <211> 8777
   <212> DNA
   <213> Clostridium cellulolyticum
<400> 7
   ctattgatgc actagattgt gccgttttca agaaacttct gttaggtatt atttaaaaat    60
   tacccgggta gaactatggt attttagtcc atatatggtt taaattgtta tatctgaatt   120
   tgtaaattgg ttaaggaggc aaacatgtta aaaactatga gtatacttct accatgctta   180
   ttgatatttt cacttatatt cagtgtacaa atacctttat cagcttcagc agcaaatgtt   240
   gaactcctaa agcagttcga catggaacag gtaaaaataa cagatacata ttatgtaaat   300
   gcacttaata aagaggttgc ctacttgcag gcaattgatc caaaccgttt gttggtgggt   360
   tttaagaaaa cagctggctt atcaacaact tatagctatt atggagggtg ggaaaacaat   420
   accctgattc aaggccatac catgggacat tacatgtcgg cacttgctca ggcttataaa   480
   aacactaagt ccgacccgac agtaaatgca gatttgaaaa gccgtatcga tttgattata   540
   tccgaattgc aggcttgtca gaataaaaac ggcaatggat atttgtttgc aactccggct   600
   acccaatttg atgttgttga aggaaaggcg tccggttcaa gctgggtacc gtggtatacc   660
   atgcacaaaa tcatgtccgg tcttcttgac atttataaat ttggaggcaa ccaaaccgca   720
   ttgacaatag caaccaactt gggaaattgg atttacaaaa gagtaaacgc ttgggattct   780
   gcaacacagt caagggtatt gggtgttgag tatggaggaa tgaatgactg tctctatgaa   840
   ttgtataagc tgactggtaa tggcaaccat ttaacagcag cacataaatt tgacgaaaat   900
   tcactattta acaccatcgc tgcaggcaca aacgttttac ccggaaaaca tgccaataca 960
   actatcccga aattcatcgg tgctttgaat cgctacagca ctctaggaac atcagaatca 1020
   tcatacttaa aagcggcaca gcagttctgg gccatagttt tgaaagacca tacatatgta 1080
   acagggggca acagcgaaga tgagcgtttc agggacgctg gcaaactgga tgcatacagg 1140
   gataatgtaa ataatgaaac ttgtaatgta aataatatgc tgaagctgac taaagagctg 1200
   ttcaaggcaa cgggcgacgt taaatatgca gattactatg agaatgcatt gataaacgaa 1260
   atcatggctt cacagaatcc ggaaaccggg atggctacgt acttcaaggc tatgggaact 1320
   ggatatttca aggtattcag ttcccaattc aatcatttct ggtgctgtac gggaacggga 1380
   atggagaatt tcacaaagct gaatgacagc ctgtattata ataatggttc cgacctgtat 1440
   gtaaacatgt atctgagttc taccctgaac tggagcgaaa agggtctttc actgacacag 1500
   caggccaatc tgccattatc agataaagta acctttacta tcaacagtgc ttcttcatca 1560
   gaagtgaaaa ttaaattcag gtcaccagca tggattgctg caggacaaaa tattacggtc 1620
   aaagttaacg gtactccaat taatgttgac aaggcgaatg gctatcttga cgtcagcaga 1680
   gtgtggcaga caggagatac ggttgagttg accctgccca ccgaagtaag ggtatccaga 1740
   ctgactgaca gccccaatac ggtagccttt acatatggtc ccgtagtatt gagtgcaggt 1800
   cttggaactg aaagcatgac aactcaatca cacggggtcc aggttttaaa agcaacgaaa 1860
   aatgtgacta tcaaagagac tattaatatt aataccgccg ccagtcccag cattgacaat 1920
   tggcttgcca atataaagaa caatttggtt caaacgcctg ggaagctgga atttacattg 1980
   aaaaataccg acgaggataa ccatttggta ttcacacctc attatcaaag atacaaggac 2040
   aggtatggta tctacttcaa gctgggaacg tatgagggta aacaacccac ggataatttg 2100
   cttgacaatc cggatattga gtcagggaac accacaggat ggactgtgaa tggtgcgggt 2160
   acaattgcct cttcaacagt acaaaagcac tcgggaagct atagtctgct gcatacaggc 2220
   aggacaggag cctggaacgg gcctattcag aacattacaa caaaagttca gaatggtaac 2280
   acgtatactt gttccggctg ggtaatactg gacaacactg ccagtgcccc gataacaatg 2340
   actatcagaa aaacggatga caacggaact tcctatgtca atattgccac tgctaccgga 2400
   agcaatagtt cctgggttca attgtcaggt aactatacct taaatgttac aggtgcattg 2460
   actgacctga gtatatattt tgaaggaccg gacagcggca ccaattttta tgtggatgat 2520
   gccttagtta aggtttatgg caaaactacc ttctatcaga atacttcttt tggcggtact 2580
   gcggtgtcgc tgaatccagg cagctatact actgctcagc tcactgctgc aggtatttct 2640
   gataactggg catcatcaat caaaatacct gaaggctata cggttgagat ttatgatgat 2700
   gacaatttca ctggtacaaa gtggtctttt agtgcagata attcgaactt tatagaagcc 2760
   ggatgcaatg acaaaatgtc ttccgtgaaa attttcccca ctctgagtca agtgaagtat 2820
   ggggatatta acagggacgg tactgtagat actattgact ttgcactttt aaagcagttt    2880
   ttgttgggtg ctcaggtcac aattgattcg gtagcggctg atttggacgg cgatgaatct    2940
   gtgacggcaa tggattttgc ggtatttaag aagtatctgc tgggacaaat aacagagttg    3000
   cctgcttttt gatgttccga tggaattatt tgattccaaa tttattgata tagcaaatca    3060
   gccgattcgt tctggtttta gaacagtcta aataacgaaa atttaggggg gataaataga    3120
   atgaaaaaag ttcgtacagt cagtacaatt atctttttag gacttatgat taccgtaatt    3180
   atggttttga atactggtgc atgggataac ggtcttgcaa aaacaccacc gatgggatgg    3240
   aacagttgga acatattcca tggagacatt aatgaaacta aaatcaaaca gattgctgat    3300
   accatggtaa gctcgggtat gaaggaagct ggctatgtat acctgaatct ggatgataac    3360
   tggatggcaa atcctgcaag agattccaat gggaacttgc gggccgatcc tacacgattc    3420
   ccaagtggga ttagggcttt agctgattat gtacatgcaa aaggtctcaa actagggata    3480
   tacggatgtc gtggaacaat gacctgtatg aatattcctc aaagcggaag caagggttat    3540
   gaggacaagg atgcaaagac atttgcttca tggggaattg attaccttaa atatgataac    3600
   tgcaatatac ctaacggaag tgacatgaaa accgattacc agaaaatgca gaccgctctt    3660
   gcaaattgcg gaagaccaat agtattcagt atatgtgcat ggggatatca gagctggatg    3720
   cctgcaacag gtaatttatg gcgtactacc ggggatttcg ctgataagtg ggataacgga    3780
   aacgaatggt tcaaaggtat tataaatgca attgatggta atgcacaata cacaagttca    3840
   gccgcacccg gtgcatggaa tgatcctgat atgcttgaaa tcggaaacgg tggatgtaca    3900
   acagaggaat accgtacaca gatgagtatg tggagtatga tggcttctcc ccttattgca    3960
   ggaaatgata taaggaccat gtcacagaca acaaaggata ttctattgaa taaggaagta    4020
   atagcaatag accaggatcc tgcaggagtt cagggaaaaa gagttaaaag tgcaaatggt    4080
   cttgagattt gggtaaaacc actgggtacg aatggtacaa ctaaggcagt tgctttattg    4140
   aacaggaatt cggcaacatc caatattaca gttaattggt cagatatagg tgtaagtgga    4200
   agtgttacgg tcagggattt gtgggctaaa tctgacaaag gcagttttac gggctcatac    4260
   acagcgtctg ttccttcaca tggaactgtt ttgattaaga tttccactga gccgccggca    4320
   cctgttgatg caacaaagca aatagaagca gagagttata gcaatcagtc aggaatccag    4380
   acagaaacct gttcggaagg cggagaggat gtaggcttta tcgaaaacgg ggactatact    4440
   gtttacagca ttgtggattt cggcgatgga gtcggaggct tccaggcaag agtagcaagt    4500
   gcgaccagcg gaggcaatat tgagattaga cttgacagcc ctgccgggac tttaattgga    4560
   acctgtccgg ttgccggaac aggggattgg cagacttata ctgatgtaaa atgtactgtc    4620
   agcggggcaa caggaaaaca tgatgtatac cttgtattta aaggagatag cggatattta    4680
   ttcaacctta attggttcac atttactcca ggaagtgtca atacgggtac attgggtgat 4740
   ttaaattccg acggacaagt agacgcgata gatttacagt tattgaaaaa gtatatttta 4800
   ggactgggag caatcgaaaa tacaaaactg gcagatttgg atgccaacgg agatatcaat 4860
   gcaatagatt tttcactgct gaaacaattc ttactaggca taaggaccag ctttccgggg 4920
   cagggggcag cataatttag cgatatcctg ataggaggtt gaaaatgaaa agaaagattt 4980
   tatctgttct tttgcttgtc acaatgacta cagcattgtt ttcagctaca ccgatgaata 5040
   ccgcttcagc cgcaagtacg gactttgtac tagacggaaa caatatcaaa gcgggcaaca 5100
   tcaacggtct cacatttaag gggttcggcg tcctcagtgg aaacagctca agtgcactgt 5160
   tgatggatta caagtcggag catcctgaga aatatacaga attactgcaa attctgttcg 5220
   gtggaaaaaa tccgattatg acacatgtca aaattgagat gggtaatgac cgtaacaact 5280
   ccaccggacc agatccctca acaatgcgtt gggaaaatga gacggctaat gtcaaaagac 5340
   accccggatt tcaacttgcg gctgatgcca agaaggtgaa ccccaatctt aaagtcagca 5400
   tattacgctg gaatgccccc ggttgggcaa atagcaatga taagatttat acatggtata 5460
   agaacaccat attagcagca taccgtcaat atggttatat gattgattac gtaaacccgg 5520
   gggtcaacga acaaacaccg aatttaacct ggactaagca atacgcccag cgtatcaaaa 5580
   cagacagtac aggttttaac aatgctgaag aacgggcact ttacaacaat attaaggtgg 5640
   tgatttccga cgaagtttcc gtcggttcct tcggggatga tatggtcagt gattccaccc 5700
   ttcgtgatgc cgtatctgtc gctgcatatc actataatac tgatgacaat agcttgggaa 5760
   gtttcaaaca gcttgccgaa tcctttgaca aagaggtgtg gaatagtgaa gcacaggcca 5820
   cttttagtaa ttcgtccttt cgtcccaata acaatatgaa agatccaaca gtagcaggaa 5880
   ccggcatagg aggcacaaat gggccactgg aaatgggaaa tactgttata aaggggtttg 5940
   taaattcaag gaggacacat tttatctatc agccagtcat tgggtccttc tatgagggtg 6000
   ggcagtactc ttttaaggaa ttggtatccg cacgtgatcc ttggtccggg tggattcact 6060
   atgatgccgg tcttgtcata cttcggcatt tcagttggtt ttcaaaggct ggctgggaaa 6120
   atgagagtaa taccgcaggg gtttggaggg ctgtacctca ggcgagtttc acaggtgcga 6180
   cgggtacgaa tcctgttaat gggcgtaatg gcactcccag ttatatgaca ctagcttctc 6240
   cggacaagca tgatttttca actatcttca ttaacgatag tgaatattcc aaaacctaca 6300
   cgtttaagac catcaatatg gcttattcgg gaaacccctc gctggaagta tgggaaacta 6360
   gagcagctga taagggagca tcttttaata gtaattacat gaagtacact ggaacggttt 6420
   ccacaaatag cagcggagtt tatacagtaa acgtaaaacc ttattcgatt gttacagtta 6480
   caacattaag taacagtgga aaagcagagt tcaatacgcc tcttccggtg gagggggacc 6540
   gcccggttct ggatacagac aagacaggtt caatgcagga taccagggat aatatattat 6600
   atgctgacaa ctttgattat tccagcaaaa ctgcccctgt catagcggag gggggacaaa 6660
   tcacaggaac ccaaagttat atagattccc gaggaggctc aaaaagtgct ataccacgtt 6720
   atgccagcga cagaaacggt gcatttgaag tgtatcttcc tgacgggtcc agcaactata 6780
   tccttcgcca acaggtgaat cagtcaagta tggggcttgg gggcacatgg aataatggta 6840
   atccaatcac cggcattgga gataaccgtt ggatgaacta taaggcaagt gtggatgttt 6900
   catttgaaca caacagtaca gagggcggta acaattatgc tgcaatcggt gccagacagc 6960
   agggtggtga aaattcacac tacttaaatg gtactcctta tatactgaaa ttctggtttg 7020
   acggcggttg gtcgctacta gtaaatggaa gttccgtggc aaatggtaat gtagcaagcg 7080
   gctcgggtgg agtgaaaatc agtggtttta atacagctta taatgcatgg cataacatct 7140
   ctatcatggt tgcagacaat aaggtgactg cgtatctgga caataccatc ctttatacct 7200
   atacagatac tacccaaaga ttgtccgggc gtattgatct ggcaagcggc tattacaata 7260
   cttgttttga caatttgaag gttgaaacaa tagacggtta tgcaccttac tactctgaaa 7320
   tgctggacaa tctggaaatg tacgatttgt cttctgtttc tgctacaaag cttgtttacg 7380
   gcggttcttg ggcacatgaa aacggcaaat ccatgtacaa ttaccaacga tcactttcca 7440
   cgagccaggg aataggtgct actattcagt attcattcac tggcaccggg ctggacattc 7500
   ttggagccaa caacggttct gctaagttag aggtaactgt tgatggaaga gttgttaatt 7560
   cctcagtggg aaccatggtt tcagggaatt tacaccaaaa ctttacgctt cacggtcttg 7620
   agtacggtaa gcatacggtt tgtttgaagg tgttaagtgg tactatggtt gtcgatgctg 7680
   ttggggttgt tgcaaacata gccggtgctt cggagatacc cgttgaacaa tctgcgtatt 7740
   caaggataga agcagagagc tacagcaacc agtcaggaat ccagacagaa acctgttcgg 7800
   aaggcggaga ggatgtgggc tttgttgaaa acggcgacta tactgtttac aacaatgtgg 7860
   atttcggcga tggtgtcgga ggcttccaag caagagtagc aagtgcaacc agtggaggca 7920
   atattgagat caggcttgac agctctaccg ggactttgat aggaacttgt cctgttgccg 7980
   gaacagggga ttggcagact tatactgatg caaaatgtac tgtcagcggg gtaacaggaa 8040
   aacatgatgt ataccttgta tttaaaggag atagcggata tttatttaat cttaactggt 8100
   ttacatttag tgagaaaact gtcataggga atttgggtga tataaactcg gacggacaag 8160
   tagatgcaat agatttacag gtattgaaaa agtatctttt gcaactaggg gaaattggag 8220
   atacgaagct ggcagatttg gatgccaacg gagaaattaa cgcaatcgat ttttcattac 8280
   tcaaacaatt tttactgggt actattatta gttttccggg agaagcacta taaggggaca 8340
   tcaccgttcc atatgtgtga agaattacaa atataatata aggaggcttt tttggtatgt 8400
   tgagaaagaa aattttatgt atatttcttg tgactgtatt aatgctaact atattaccaa 8460
   tacctcaaca gacggtaatg gctgatacag gggtacttaa ggaacttaaa ggaactgaca     8520
   tctacaacgg attaagggga ctgaatttta acgagggttg gaaattcaat aagggggatg     8580
   taagcaacgg ccagagtacc gggtataatg acagcggctg gtcaggtgtt acactgccac     8640
   atgactggag tatttataac acttttaata aatcctcagc agcgggtgca ggaggaggtt     8700
   atctggatgg aggaatcgga tggtacagga aaacctttac cgtaccttcg gattatacag     8760
   gaaagaaggt attcatt                                                    8777
<210> 8
   <211> 604
   <212> PRT
   <213> Clostridium cellulolyticum
<400> 8
   Met Lys Lys Val Arg Thr Val Ser Thr Ile Ile Phe Leu Gly Leu Met
   1               5                   10                  15
   Ile Thr Val Ile Met Val Leu Asn Thr Gly Ala Trp Asp Asn Gly Leu
               20                  25                  30
   Ala Lys Thr Pro Pro Met Gly Trp Asn Ser Trp Asn Ile Phe His Gly
           35                  40                  45
   Asp Ile Asn Glu Thr Lys Ile Lys Gln Ile Ala Asp Thr Met Val Ser
       50                  55                  60
   Ser Gly Met Lys Glu Ala Gly Tyr Val Tyr Leu Asn Leu Asp Asp Asn
   65                  70                  75                  80
   Trp Met Ala Asn Pro Ala Arg Asp Ser Asn Gly Asn Leu Arg Ala Asp
                   85                  90                  95
   Pro Thr Arg Phe Pro Ser Gly Ile Arg Ala Leu Ala Asp Tyr Val His
               100                 105                 110
   Ala Lys Gly Leu Lys Leu Gly Ile Tyr Gly Cys Arg Gly Thr Met Thr
           115                 120                 125
   Cys Met Asn Ile Pro Gln Ser Gly Ser Lys Gly Tyr Glu Asp Lys Asp
       130                 135                 140
   Ala Lys Thr Phe Ala Ser Trp Gly Ile Asp Tyr Leu Lys Tyr Asp Asn
   145                 150                 155                 160
   Cys Asn Ile Pro Asn Gly Ser Asp Met Lys Thr Asp Tyr Gln Lys Met
                   165                 170                 175
   Gln Thr Ala Leu Ala Asn Cys Gly Arg Pro Ile Val Phe Ser Ile Cys
               180                 185                 190
   Ala Trp Gly Tyr Gln Ser Trp Met Pro Ala Thr Gly Asn Leu Trp Arg
           195                 200                 205
   Thr Thr Gly Asp Ile Ala Asp Lys Trp Asp Asn Gly Asn Glu Trp Phe
       210                 215                 220
   Lys Gly Ile Ile Asn Ala Ile Asp Gly Asn Ala Gln Tyr Thr Ser Ser
   225                 230                 235                 240
   Ala Ala Pro Gly Ala Trp Asn Asp Pro Asp Met Leu Glu Ile Gly Asn
                   245                 250                 255
   Gly Gly Cys Thr Thr Glu Glu Tyr Arg Thr Gln Met Ser Met Trp Ser
               260                 265                 270
   Met Met Ala Ser Pro Leu Ile Ala Gly Asn Asp Ile Arg Thr Met Ser
           275                 280                 285
   Gln Thr Thr Lys Asp Ile Leu Leu Asn Lys Glu Val Ile Ala Ile Asp
       290                 295                 300
   Gln Asp Pro Ala Gly Val Gln Gly Lys Arg Val Lys Ser Ala Asn Gly
   305                 310                 315                 320
   Leu Glu Ile Trp Val Lys Pro Leu Gly Thr Asn Gly Thr Thr Lys Ala
                   325                 330                 335
   Val Ala Leu Leu Asn Arg Asn Ser Ala Thr Ser Asn Ile Thr Val Asn
               340                 345                 350
   Trp Ser Asp Ile Gly Val Ser Gly Ser Val Thr Val Arg Asp Leu Trp
           355                 360                 365
   Ala Lys Ser Asp Lys Gly Ser Phe Thr Gly Ser Tyr Thr Ala Ser Val
       370                 375                 380
   Pro Ser His Gly Thr Val Leu Ile Lys Ile Ser Thr Glu Pro Pro Ala
   385                 390                 395                 400
   Pro Val Asp Ala Thr Lys Gln Ile Glu Ala Glu Ser Tyr Ser Asn Gln
                   405                 410                 415
   Ser Gly Ile Gln Thr Glu Thr Cys Ser Glu Gly Gly Glu Asp Val Gly
               420                 425                 430
   Phe Ile Glu Asn Gly Asp Tyr Thr Val Tyr Ser Asn Val Asp Phe Gly
           435                 440                 445
   Asp Gly Val Gly Gly Phe Gln Ala Arg Val Ala Ser Ala Thr Ser Gly
       450                 455                 460
   Gly Asn Ile Glu Ile Arg Leu Asp Ser Pro Ala Gly Thr Leu Ile Gly
   465                 470                 475                 480
   Thr Cys Pro Val Ala Gly Thr Gly Asp Trp Gln Thr Tyr Thr Asp Val
                   485                 490                 495
   Lys Cys Thr Val Ser Gly Ala Thr Gly Lys His Asp Val Tyr Leu Val
               500                 505                 510
   Phe Lys Gly Asp Ser Gly Tyr Leu Phe Asn Leu Asn Trp Phe Thr Phe
           515                 520                 525
   Thr Pro Gly Ser Val Asn Thr Gly Thr Leu Gly Asp Leu Asn Ser Asp
       530                 535                 540
   Gly Gln Val Asp Ala Ile Asp Leu Gln Leu Leu Lys Lys Tyr Ile Leu
   545                 550                 555                 560
   Gly Leu Gly Ala Ile Glu Asn Thr Lys Leu Ala Asp Leu Asp Ala Asn
                   565                 570                 575
   Gly Asp Ile Asn Ala Ile Asp Phe Ser Leu Leu Lys Gln Phe Leu Leu
               580                 585                 590
   Gly Ile Arg Thr Ser Phe Pro Gly Gln Gly Ala Ala
           595                 600

## Description

### Background of the Invention

Various tissue-derived products are used to regenerate, repair, or otherwise treat diseased or damaged tissues and organs. Such products can include intact tissue grafts and/or acellular or reconstituted acellular tissues (*e.g.,* acellular tissue matrices from skin, intestine, or other tissues, with or without cell seeding), which may be derived from a donor of a different species from the recipient (xenograft) or from a donor of the same species as the recipient (allograft). For example, a collagen-containing material may be made from porcine tissue and implanted in a human patient.

In recipient animals (*e.g.,* humans) that do not express the enzyme β-D-galactosyl-1,4-N-acetyl-D-glucosaminide α-1,3 galactosyl-transferase (α-1,3 galactosyltransferase; "αGT"), which catalyzes the binding of α-1,3galactose (Gal) on N-acetyllactosamine (Galβ1,4GlcNAc) to produce terminal Galα1,3Galβ1,4GlcNAc-R ("galactose-alpha-1,3-galactose," "α-gal epitope," or "α-gal") on an acceptor substrate, a major problem of xenotransplanted tissue is hyperacute rejection of xenografts in such recipients that is largely, if not exclusively, due to the action of antibodies specific for the α-gal epitope on the surface of cells in the xenograft.

The α-gal epitope is expressed in non-primate mammals and in New World monkeys (monkeys of South America) as well as on macromolecules such as proteoglycans of the extracellular components, but is absent in Old World primates (monkeys of Asia and Africa and apes) and humans (U. Galili et al.(1988) J. Biol. Chem. 263: 17755). Anti-gal antibodies are produced in humans and primates as a result of an immune response to α-gal epitope carbohydrate structures on gastrointestinal bacteria. U. Galili et al. (1988) Infect. Immun. 56:1730; R. M. Hamadeh et al.(1992) J. Clin. Invest. 89:1223.

Since non-primate mammals (*e.g.,* pigs) produce α-gal epitopes, xenotransplantation of tissue material from these mammals into primates often results in rejection because of primate anti-Gal antibody binding to these epitopes. The binding results in the destruction of the tissue material by complement fixation and antibody dependent cell cytotoxicity. U. Galili et al. (1993) Immunology Today 14:480; M. Sandrin et al. (1993) Proc. Natl. Acad. Sci. USA 90:11391; H. Good et al.(1992) Transplant. Proc. 24:559; B. H. Collins et al. (1995) J. Immunol. 154: 5500. Accordingly, when animals that produce α-gal epitopes are used as the tissue source for treatment of diseased or damaged tissue or organs, the removal of α-gal epitopes from cells and from extracellular components of the tissue material can diminish the immune response associated with anti-gal antibody binding to α-gal epitopes.

Enzymes such as alpha-galactosidases from green coffee beans and *Bacteroides,* which substantially eliminate α-gal epitopes from cells and from extracellular components of a collagen-containing material, have been identified and used to prepare tissue products (see, *e.g.,* Luo, et al. (1999) Xenotransplantation 6(4):238-48; U.S. Patent No.:7,951,552). However, supply issues have decreased the availability of these enzymes, thereby increasing the cost and availability of tissue products to treat subjects in need thereof. Accordingly, there is a need for additional enzymes and methods for treating tissue products to reduce or control the immune response of the tissue products upon implantation.

WO99/12555A discloses a tissue enzymatically treated with α-galactosidase to remove the Gal epitope. US 7,951,552 B discloses a method of preparing a non-human tissue for xenotransplantation, comprising the step of incubating the non-human tissue with an enzyme having α-galactosidase activity.

The present invention is defined in the claims. Any references in the description to methods of treatment refer to the compounds, pharmaceutical compositions and medicaments of the present invention for use in a method for treatment of the human (or animal) body by therapy (or for diagnosis). In one aspect, the present disclosure provides methods for preparation of a non-human tissue matrix for xenotransplantation. The methods can include contacting a collagen-containing tissue matrix with an isolated *Trichoderma reesei* or *Clostridium cellulyticum* alpha-galactosidase in an amount and for a time sufficient to remove an α-gal epitope from the tissue matrix, thereby preparing the non-human tissue matrix for xenotransplantation.

In one embodiment, the alpha-galactosidase comprises an amino acid sequence having at least 85% identity to the entire amino acid sequence set forth in a sequence selected from the group consisting of SEQ ID NOs:2, 4, 6, and 8. In another embodiment, the alpha-galactosidase is encoded by a nucleic acid molecule comprising a nucleotide sequence having at least 85% identity to the entire nucleotide sequence set forth in a sequence selected from the group consisting of SEQ ID NOs:1, 3, 5, and 7. In another embodiment, the alpha-galactosidase is encoded by a nucleic acid molecule, said nucleic acid molecule encoding a protein comprising an amino acid sequence having at least 85% identity to the entire amino acid sequence set forth in a sequence selected from the group consisting of SEQ ID NOs:2, 4, 6, and 8.

In one embodiment, the tissue matrix is an acellular tissue matrix. In another embodiment, the tissue matrix comprises a dermal tissue matrix.

In one embodiment, the tissue matrix is obtained from a tissue selected from fascia, pericardial tissue, dura, umbilical cord tissue, placental tissue, cardiac valve tissue, ligament tissue, tendon tissue, arterial tissue, venous tissue, neural connective tissue, urinary bladder tissue, ureter tissue, and intestinal tissue.

In one embodiment, the methods of the invention further comprise treating the tissue matrix to remove at least some of the cells and cellular components, *e.g.,* substantially all the cells and cellular components, from the tissue matrix.

In one embodiment, the methods of the invention further comprise treating the tissue matrix to remove substantially all cells and cellular components prior to contacting the tissue matrix with the alpha-galactosidase.

In one embodiment, the at least one collagen-containing tissue matrix includes two or more tissue matrices.

In one embodiment, the methods of the invention further comprise packaging the tissue matrix.

In one embodiment, the methods of the invention further comprise sterilizing the tissue matrix.

In another aspect of the disclosure, the present disclosure provides a tissue matrix prepared according to the method of the invention.

### BRIEF DESCRIPTION OF THE DRAWINGS

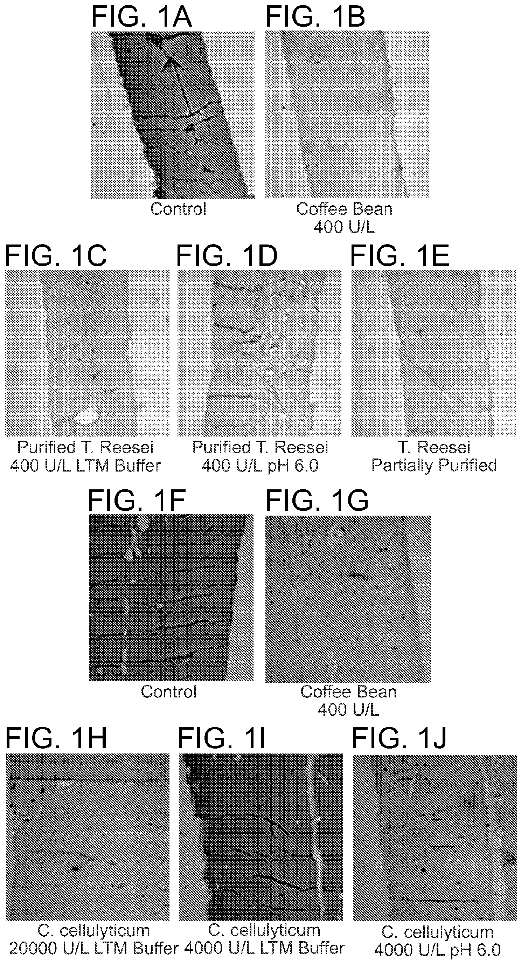

Figures 1A-1J show acellular tissue matrices stained for the α-gal epitope. Figures 1C-1E and 1H-1J show acellular tissue matrices stained for the α-gal epitope following treatment with the indicated enzymes using the methods described in Example 1. Figures 1A and 1F show untreated control acellular tissue matrices consisting of porcine dermis that was not exposed to enzyme and stained for the α-gal epitope, and Figures 1B and 1G are positive controls consisting of porcine dermis treated with green coffee bean alpha-galactosidase. The presence of α-gal epitope is indicated by a dark gray color.

### DESCRIPTION OF EXEMPLARY EMBODIMENTS

The present invention is defined in the claims. The present invention is based, at least in part, on the discovery of microbial alpha-galactosidases that remove terminal alpha-1,3 linked galactose residues from animal tissue and methods of using such alpha-galactosidases for preparing a non-human tissue matrix that may be used, *e.g.,* as a tissue product. As described in the appended examples below, it has been discovered that of the multitude of microbial alpha-galactosidases that have been identified, only alpha-glactosidases from *Trichoderma reesei* and *Clostridium cellulolyticum* effectively cleave α-gal epitopes from non-human tissue and, thus, *e.g.,* reduce the immunogenicity of tissue matrices prepared for xenotransplantation.

Accordingly, the present disclosure provides methods for preparing non-human tissue or tissue matrices for implantation into human patients.

### I. Definitions

Definitions of certain terms are first defined below. In addition, it should be noted that whenever a value or range of values of a parameter are recited, it is intended that values and ranges intermediate to the recited values are also intended to be part of this invention.
The articles "a" and "an" are used herein to refer to one or to more than one (*i.e.,* to at least one) of the grammatical object of the article. By way of example, "an element" means one element or more than one element, *e.g.,* a plurality of elements.
The term "including" is used herein to mean, and is used interchangeably with, the phrase "including but not limited to".
The term "or" is used herein to mean, and is used interchangeably with, the term "and/or," unless context clearly indicates otherwise.
As used herein, the term "subject" refers to human and non-human animals, *e.g.,* veterinary subjects. The term "non-human animal" includes all vertebrates, *e.g.,* mammals and non-mammals, such as non-human primates, mice, rabbits, sheep, dog, cat, horse, cow, chickens, amphibians, and reptiles. In one embodiment, the subject is a human.
As used herein "tissue product" refers to any tissue that contains extracellular matrix proteins. Various tissues can be used to produce tissue products for, *e.g.,* treating a subject in need thereof. For example, various tissue products for regeneration, repair, augmentation, reinforcement, and/or treatment of human tissues that have been damaged or lost due to various diseases and/or structural damage (*e.g.,* from trauma, surgery, atrophy, and/or long-term wear and degeneration) have been produced. Such products can include, for example, acellular or partially decellularized tissue matrices, decellularized tissue matrices that have been repopulated with exogenous cells, and/or cellular tissues.

The tissues can be selected from a variety of tissue sources including skin (dermis or whole skin), fascia, pericardial tissue, dura, umbilical cord tissue, placental tissue, cardiac valve tissue, ligament tissue, adipose tissue, tendon tissue, arterial tissue, venous tissue, neural connective tissue, urinary bladder tissue, ureter tissue, and intestinal tissue. The methods described herein can be used to process any collagenous tissue type, and for any tissue matrix product. For example, a number of biological scaffold materials are described by Badylak et al. (Acta Biomaterialia (2008), doi:10.1016/j.actbio.2008.09.013), and the methods of the present disclosure can be used to treat those or other tissue products known in the art.

In some cases, the tissue products can be provided as decellularized tissue matrices. Suitable acellular tissue matrices are described further below. In some cases, the methods of the may further include processing intact tissue to remove cells or other materials. The tissues can be completely or partially decellularized to yield acellular tissue matrices or extracellular tissue materials. For example, various tissues, such as skin, intestine, bone, cartilage, adipose tissue, nerve tissue (*e.g.,* nerve fibers or dura), tendons, ligaments, or other tissues can be completely or partially decellularized to produce tissue products useful for patients. In some cases, these decellularized products can be used without addition of exogenous cellular materials (*e.g.,* stem cells). In certain cases, these decellularized products can be seeded with cells from autologous sources or other sources to facilitate treatment. Suitable processes for producing acellular tissue matrices are described below.

### II. Methods of Preparing Tissue Products

The present disclosure provides methods for preparation of a non-human tissue matrix for xenotransplantation. The methods can include contacting a collagen-containing tissue matrix with an isolated *Trichoderma reesei* or *Clostridium cellulyticum* alpha-galactosidase in an amount and for a time sufficient to remove an α-gal epitope from the tissue matrix, thereby preparing the non-human tissue matrix for xenotransplantation. A "sufficient amount and time to remove an α-gal epitope from the tissue matrix" is the amount of the enzyme and the time that the tissue matrix is contacted with the enzyme to reduce the immunogenicity of the tissue matrix prepared for xenotransplantation. An immune response can be measured using a number of immunoassays, including monocyte activation assays, phagocytosis assays, and/or oxidative burst assays, which are readily known to one of ordinary skill in the art.

The term "reduce" with respect to the immunogenicity of a tissue matrix refers to a statistically significant decrease in such level. The decrease can be, for example, at least 10%, at least 15%, at least 20%, at least 25%, at least 30%, at least 35%, at least 40%, at least 45%, at least 50%, at least 55%, at least 60%, at least 65%, at least 70%, at least 75%, at least 80%, at least 85%, at least 90%, at least 95%, or more.

Any suitable enzyme concentration, buffer, pH, temperature, and treatment time can be used as long as it is sufficient to remove an α-gal epitope from the tissue matrix. In some embodiments, the conditions suitable for treating a tissue sample as described herein may include about 50 U/L to about 400 U/L of an α-galactosidase in a buffer at a physiologically acceptable pH (*e.g.,* about pH 6.0 to about pH 8.0) and temperature (*e.g.,* about 20°C to about 40°C), *e.g.,* about 50, 60, 70, 80, 90, 100, 110, 120, 130, 140, 150, 160, 170, 180, 190, 200, 210, 220, 230 240, 250, 260, 270, 280, 290, 300, 310, 320, 330, 340, 350, 360, 370, 380, 390, or about 400 U/L an α-galactosidase in a, *e.g.,* phosphate buffer, having a pH of about 6.0, 6.1, 6.2, 6.3, 6.4, 6.5, 6.6, 6.7, 6.8, 6.9, 7.0, 7.1, 7.2, 7.3, 7.4, 7.5, 7.6, 7.7, 7.8, 7.9, or about 8.0, at about 20°C, 21°C, 22°C, 23°C, 24°C, 25°C, 26°C, 27°C, 28°C, 29°C, 30°C, 31°C, 32°C, 33°C, 34°C, 35°C, 36°C, 37°C, 38°C, 39°C, or 40°C. Ranges and values intermediate to the above recited ranges and values are also contemplated to be part of the invention. In an exemplary embodiment, the tissue sample is treated with an α-galactosidase at a concentration of 300 U/L prepared in 100 mM phosphate buffer at pH 6.0. In other embodiments, the concentration of α-galactosidase is increased to 400 U/L for adequate removal of the α-gal epitopes from the harvested tissue.

Species that can serve as recipients of a tissue matrix and donors of tissues or organs for the production of the tissue matrix include, without limitation, mammals, such as humans and non-human primates (*e.g.,* monkeys, baboons, or chimpanzees).

In one embodiment, the methods further include at least partial decellularization of the tissue. The decellularization step may be performed before contacting the tissue with the glactosidase or concomitantly with contacting the tissue with the galactosidase. The tissue may be a dermal tissue.

The present disclosure also provides methods of treating a subject with a tissue matrix of the present invention. The methods can include preparing a tissue matrix as described herein, identifying a mammalian subject as having an organ, or tissue, in need of repair or amelioration; and placing the tissue matrix in or on the organ or tissue. In one embodiment, the subject is human. The methods can further comprise administration to the subject of one or more agents, *e.g.,* a cell growth factor, an angiogenic factor, a differentiation factor, a cytokine, a hormone, or a chemokine. The one or more agents can be in the tissue matrix placed in the subject or they can be injected or infused into the subject separately from the tissue matrix. The organ or tissue of the subject can be, without limitation, skin, bone, cartilage, meniscus, dermis, myocardium, periosteum, artery, vein, stomach, small intestine, large intestine, diaphragm, tendon, ligament, neural tissue, striated muscle, smooth muscle, bladder, urethra, ureter, gingival, or fascia (*e.g.,* abdominal wall fascia).

As used herein, the terms "treating" or "treatment" refer to a beneficial or desired result including, but not limited to, alleviation or amelioration of one or more symptoms. "Treatment" can also mean prolonging survival as compared to expected survival in the absence of treatment.

As used herein, the term "placing" a tissue matrix includes, without limitation, setting, injecting, infusing, pouring, packing, layering, spraying, and encasing the composition. In addition, placing "on" a recipient tissue or organ means placing in contact with the recipient tissue or organ.

Suitable *T. reesei* and *C. cellulolyticum* alpha-galactosidases for use in the methods of the present invention may be either naturally occurring (native) or genetically engineered. For example, suitable enzymes may be obtained by, for example, fermentation of the organisms and use of an appropriate purification scheme using standard protein purification techniques. For example, cell supernatants may be collected and concentrated (*e.g.,* by ultrafiltration) and applied to, for example, an isoelectric focusing matrix to identify fractions having alpha-galactosidase activity. Active fractions may be further purified using, for example, ion-exchange chromatography and/or gel filtration. Alternatively, recombinant DNA techniques may be used to produce a *T. reesei* and *C. cellulolyticum* alpha-galactosidase comprising the whole or a segment of the protein (a functional fragment of the protein). For example, recombinant DNA techniques may be used to clone a nucleotide sequence encoding a segment or the whole protein into a vector (such as an expression vector) and transform a cell for production of the protein. A *T. reesei* and *C. cellulolyticum* alpha-galactosidase comprising the whole or a segment of the protein may also be synthesized chemically using standard peptide synthesis techniques.

The nucleotide and amino acid sequences of *T. reesei* and *C. cellulolyticum* alpha-galactosidases may be found in, for example, GenBank (see, *e.g.,* www.ncbi.nlm.nih.gov) or UniProt (see, *e.g.,* www.uniprot.org/uniprot). In particular, *T. reesei* has three genes (*aglI, aglII,* and *aglIII*) that encode proteins having alpha-galactosidase activity for use in the methods of the invention, the nucleotide and amino acid sequence of which may be found in, for example, GenBank Accession numbers GI:1580815 (SEQ ID NO:1); GI:74630547 (SEQ ID NO:2); GI:1580817 (SEQ ID NO:3); GI:74630548 (SEQ ID NO:4); GI:1580811 (SEQ ID NO:5); and GI:74630544 (SEQ ID NO:6). It should be understood that any one, two or three of the *T. reesei* nucleic acid molecules or proteins may be used in the methods of the invention. *C. cellulolyticum* has a single alpha-galactosidase gene encoding a protein having alpha-galactosidase activity for use in the methods of the invention, the nucleotide and amino acid sequence of which may be found in, for example, GenBank Accession numbers GI:110588919 (nucleotides 3121-4935 of SEQ ID NO:7); and GI:219998992 (SEQ ID NO:8).

As used herein, the terms an "isolated molecule", such as an "isolated nucleic acid molecule", "an isolated polypeptide", "an isolated protein", is one which is separated from other molecules which are present in the natural source of the molecule. In one embodiment, an "isolated nucleic acid molecule", is free of sequences (such as protein-encoding sequences) which naturally flank the nucleic acid (*i.e.,* sequences located at the 5' and 3' ends of the nucleic acid) in the genomic DNA of the organism from which the nucleic acid is derived. For example, in various embodiments, an isolated nucleic acid molecule can contain less than about 5 kB, 4 kB, 3 kB, 2 kB, 1 kB, 0.5 kB or 0.1 kB of nucleotide sequences which naturally flank the nucleic acid molecule in genomic DNA of the cell from which the nucleic acid is derived. In another embodiment, an "isolated" molecule can be substantially free of other cellular material, or culture medium when produced by recombinant techniques, or substantially free of chemical precursors or other chemicals when chemically synthesized. A molecule that is substantially free of cellular material includes preparations having less than about 30%, 20%, 19%, 18%, 17%, 16%, 15%, 14%, 13%, 12%, 11%, 10%, 9%, 8%, 7%, 6%, or about 5% of heterologous molecules and which retains alpha-galactosidase activity.

RNA or DNA encoding the alpha-galactosidases may be readily isolated, amplified, and/or sequenced using conventional procedures (*e.g.,* by using oligonucleotide probes that are capable of binding specifically to the relevant genes, as described in, for example, Innis et al. in PCR Protocols. A Guide to Methods and Applications, Academic (1990), and Sanger et al., Proc Natl Acad Sci USA 74:5463 (1977)). A nucleic acid molecule so amplified may be cloned into an appropriate vector and characterized by DNA sequence analysis. Furthermore, nucleotides corresponding to all or a portion of an isolated nucleic acid molecule for use in the methods of the invention can be prepared by standard synthetic techniques, *e.g.,* using an automated DNA synthesizer.

In one embodiment, an isolated nucleic acid molecule for use in the methods of the invention comprises a nucleic acid molecule which has a nucleotide sequence complementary to the nucleotide sequence of a nucleic acid molecule encoding a *T. reesei* or a *C. cellulolyticum* alpha-galactosidase. A nucleic acid molecule which is complementary to a given nucleotide sequence is one which is sufficiently complementary to the given nucleotide sequence that it can hybridize to the given nucleotide sequence thereby forming a stable duplex.

Moreover, a nucleic acid molecule for use in the methods of the invention can comprise only a portion of a nucleic acid sequence which encodes a *T. reesei* or a *C. cellulolyticum* alpha-galactosidase. Such nucleic acid molecules can be used, for example, as a probe or primer. The probe/primer typically is used as one or more substantially purified oligonucleotides. The oligonucleotide typically comprises a region of nucleotide sequence that hybridizes under stringent conditions to at least about 7, preferably about 15, more preferably about 25, 50, 75, 100, 125, 150, 175, 200, 250, 300, 350, or 400 or more consecutive nucleotides of a nucleic acid molecule for use in the methods of the invention.

The invention further encompasses nucleic acid molecules that differ, due to degeneracy of the genetic code, from the nucleotide sequence of nucleic acid molecules encoding a *T. reesei* or a *C. cellulolyticum* alpha-galactosidase protein and thus encode the same protein. It will be appreciated by those skilled in the art that DNA sequence polymorphisms that lead to changes in the amino acid sequence can exist within a population. Such genetic polymorphisms can exist among individuals within a population due to natural allelic variation. An allele is one of a group of genes which occur alternatively at a given genetic locus. In addition, it will be appreciated that DNA polymorphisms that affect RNA expression levels can also exist that may affect the overall expression level of that gene (*e.g.,* by affecting regulation or degradation).

Accordingly, in one embodiment a nucleic acid molecule suitable for use in the methods of the invention is at least about 40% identical, about 50%, 60%, 70%, 80%, 81%, 82%, 83%, 84%, 85%, 86%, 87%, 88%, 89%, 90%, 91%, 92%, 93%, 94%, 95%, 96%, 97%, 98%, or about 99% identical to the nucleotide sequence of a *T. reesei* or a *C. cellulolyticum* alpha-galactosidase.

In addition to naturally occurring allelic variants of a nucleic acid molecule of the invention that can exist in the population, the skilled artisan will further appreciate that sequence changes can be introduced by mutation thereby leading to changes in the amino acid sequence of the encoded protein, without altering the biological activity of the protein encoded thereby. For example, one can make nucleotide substitutions leading to amino acid substitutions at "non-essential" amino acid residues. A "non-essential" amino acid residue is a residue that can be altered from the wild-type sequence without altering the biological activity, whereas an "essential" amino acid residue is required for biological activity. For example, amino acid residues that are not conserved or only semi-conserved among homologs of various species may be non-essential for activity and thus would be likely targets for alteration. Alternatively, amino acid residues that are conserved among the homologs of various species may be essential for activity and thus would not be likely targets for alteration.

Accordingly, another aspect of the invention pertains to nucleic acid molecules encoding a variant protein that contain changes in amino acid residues that are not essential for activity. Such variant proteins differ in amino acid sequence from the naturally-occurring proteins, yet retain biological activity. In one embodiment, such a variant protein has an amino acid sequence that is at least about 40% identical, 50%, 60%, 70%, 80%, 81%, 82%, 83%, 84%, 85%, 86%, 87%, 88%, 89%, 90%, 91%, 92%, 93%, 94%, 95%, 96%, 97%, 98%, or 99% identical to the amino acid sequence of a *T. reesei* or a *C. cellulolyticum* alpha-galactosidase.

Identity or similarity with respect to parent amino acid sequence is defined herein as the percentage of amino acid residues in the candidate sequence that are identical (*i.e.,* same residue) or similar (*i.e.,* amino acid residue from the same group based on common side-chain properties, *supra*) with the parent molecule residues, after aligning the sequences and introducing gaps, if necessary, to achieve the maximum percent sequence identity.

To determine the percent identity of two amino acid sequences or of two nucleic acids, the sequences are aligned for optimal comparison purposes (*e.g.,* gaps can be introduced in the sequence of a first amino acid or nucleic acid sequence for optimal alignment with a second amino or nucleic acid sequence). The amino acid residues or nucleotides at corresponding amino acid positions or nucleotide positions are then compared. When a position in the first sequence is occupied by the same amino acid residue or nucleotide as the corresponding position in the second sequence, then the molecules are identical at that position. The percent identity between the two sequences is a function of the number of identical positions shared by the sequences (*i.e.,* % identity = # of identical positions/total # of positions (*e.g.,* overlapping positions) x 100). In one embodiment the two sequences are the same length.

The determination of percent identity between two sequences can be accomplished using a mathematical algorithm (see, *e.g.,* Karlin and Altschul (1990) Proc. Natl. Acad. Sci. USA 87:2264-2268; Karlin and Altschul (1993) Proc. Natl. Acad. Sci. USA 90:5873-5877; Altschul, et al. (1990) J. Mol. Biol. 215:403-410; Altschul et al. (1997) Nucleic Acids Res. 25:3389-3402; Myers and Miller, (1988) CABIOS 4:11-17; Pearson and Lipman (1988) Proc. Natl. Acad. Sci. USA 85:2444-2448).

An isolated nucleic acid molecule encoding a variant protein can be created by introducing one or more nucleotide substitutions, additions or deletions into the nucleotide sequence of nucleic acids, such that one or more amino acid residue substitutions, additions, or deletions are introduced into the encoded protein. Mutations can be introduced by standard techniques, such as site-directed mutagenesis and PCR-mediated mutagenesis. Preferably, conservative amino acid substitutions are made at one or more predicted non-essential amino acid residues. A "conservative amino acid substitution" is one in which the amino acid residue is replaced with an amino acid residue having a similar side chain. Families of amino acid residues having similar side chains have been defined in the art. These families include amino acids with basic side chains (*e.g.,* lysine, arginine, histidine), acidic side chains (*e.g.,* aspartic acid, glutamic acid), uncharged polar side chains (*e.g.,* glycine, asparagine, glutamine, serine, threonine, tyrosine, cysteine), non-polar side chains (*e.g.,* alanine, valine, leucine, isoleucine, proline, phenylalanine, methionine, tryptophan), beta-branched side chains (e.g., threonine, valine, isoleucine) and aromatic side chains (*e.g.,* tyrosine, phenylalanine, tryptophan, histidine). Alternatively, mutations can be introduced randomly along all or part of the coding sequence, such as by saturation mutagenesis, and the resultant mutants can be screened for biological activity to identify mutants that retain activity. Following mutagenesis, the encoded protein can be expressed recombinantly and the activity of the protein can be determined.

Biologically active portions of a *T. reesei* or a *C. cellulolyticum* alpha-galactosidase are also included within the scope of the present invention. Such biologically active portions include polypeptides comprising amino acid sequences sufficiently identical to or derived from the amino acid sequence of a *T. reesei* or a *C. cellulolyticum* alpha-galactosidase protein, which include fewer amino acids than the full length protein, and exhibit at least one activity of the corresponding full-length protein. Typically, biologically active portions comprise a domain or motif with at least one activity of the corresponding full-length protein. A biologically active portion of a protein for use in the methods of the invention can be a polypeptide which is, for example, 10, 25, 50, 100 or more amino acids in length. Moreover, other biologically active portions, in which other regions of the protein are deleted, can be prepared by recombinant techniques and evaluated for one or more of the functional activities of the native form of the protein (*e.g.,* removal of α-gal epitopes).

Another aspect of the disclosure provides chimeric or fusion proteins comprising a *T. reesei* or a *C. cellulolyticum* alpha-galactosidase protein or a segment thereof. As used herein, a "chimeric protein" or "fusion protein" comprises all or part (preferably a biologically active part) of a *T. reesei* or a *C. cellulolyticum* alpha-galactosidase protein operably linked to a heterologous polypeptide (*i.e.,* a polypeptide other than the alpha-galactosidase protein). Within the fusion protein, the term "operably linked" is intended to indicate that the alpha-galactosidase protein or segment thereof and the heterologous polypeptide are fused in-frame to each other. The heterologous polypeptide can be fused to the amino-terminus or the carboxyl-terminus of the alpha-galactosidase protein or segment.

Chimeric and fusion proteins of the disclosure can be produced by standard recombinant DNA techniques. In another aspect of the disclosure, the fusion gene can be synthesized by conventional techniques including automated DNA synthesizers. Alternatively, PCR amplification of gene fragments can be carried out using anchor primers which give rise to complementary overhangs between two consecutive gene fragments which can subsequently be annealed and re-amplified to generate a chimeric gene sequence (see, *e.g.,* Ausubel *et al., supra*). Moreover, many expression vectors are commercially available that already encode a fusion moiety (*e.g.,* a GST polypeptide). A nucleic acid encoding a polypeptide of the disclosure can be cloned into such an expression vector such that the fusion moiety is linked in-frame to the polypeptide for use in the methods of the invention.

A signal sequence can be used to facilitate secretion and isolation of *T. reesei* or a *C. cellulolyticum* alpha-galactosidase proteins. Signal sequences are typically characterized by a core of hydrophobic amino acids which are generally cleaved from the mature protein during secretion in one or more cleavage events. Such signal peptides contain processing sites that allow cleavage of the signal sequence from the mature proteins as they pass through the secretory pathway. Thus, the invention pertains to *T. reesei* or a *C. cellulolyticum* alpha-galactosidase proteins, fusion proteins or segments thereof having a signal sequence, as well as to such proteins from which the signal sequence has been proteolytically cleaved (*i.e.,* the cleavage products). In one embodiment, a nucleic acid sequence encoding a signal sequence can be operably linked in an expression vector to a nucleitc acid molecule encoding a protein of interest, such as a *T. reesei* or a *C. cellulolyticum* alpha-galactosidase protein, or a segment thereof. The signal sequence directs secretion of the protein, such as from a eukaryotic host into which the expression vector is transformed, and the signal sequence is subsequently or concurrently cleaved. The protein can then be readily purified from the extracellular medium by art recognized methods. Alternatively, the signal sequence can be linked to the protein of interest using a sequence which facilitates purification, such as with a poly-histidine tag, a strep-tag, a FLAG-tag, a GST domain, *etc.*

Nucleic acid molecules encoding the polypeptides, or functional fragments thereof, for use in the methods of the invention may be incorporated in suitable recombinant vectors. As used herein, the term "vector" refers to a nucleic acid molecule capable of transporting another nucleic acid to which it has been linked. One type of vector is a "plasmid", which refers to a circular double stranded DNA loop into which additional DNA segments can be ligated. Another type of vector is a viral vector, wherein additional DNA segments can be ligated into the viral genome. Certain vectors are capable of autonomous replication in a host cell into which they are introduced (*e.g.,* bacterial vectors having a bacterial origin of replication and episomal mammalian vectors). Other vectors (*e.g.,* non-episomal mammalian vectors) are integrated into the genome of a host cell upon introduction into the host cell, and thereby are replicated along with the host genome. Moreover, certain vectors, namely expression vectors, are capable of directing the expression of genes to which they are operably linked. In general, expression vectors of utility in recombinant DNA techniques are often in the form of plasmids (vectors). However, the invention is intended to include such other forms of expression vectors, such as viral vectors (*e.g.,* replication defective retroviruses, adenoviruses and adeno-associated viruses), which serve equivalent functions.

The recombinant vectors can comprise a nucleic acid encoding a polypeptide in a form suitable for expression of the nucleic acid in a host cell. In some embodiments, this means that the recombinant vectors may include one or more regulatory sequences, selected on the basis of the host cells to be used for expression, which is operably linked to the nucleic acid sequence to be expressed (*i.e.,* a recombinant expression vector). Within a recombinant expression vector, "operably linked" is intended to mean that the nucleotide sequence of interest is linked to the regulatory sequence(s) in a manner which allows for expression of the nucleotide sequence (*e.g.,* in an *in vitro* transcription/translation system or in a host cell when the vector is introduced into the host cell). The term "regulatory sequence" is intended to include promoters, enhancers and other expression control elements (*e.g.,* polyadenylation signals). Such regulatory sequences are described, for example, in Goeddel, Methods in Enzymology: Gene Expression Technology vol.185, Academic Press, San Diego, CA (1991). Regulatory sequences include those which direct constitutive expression of a nucleotide sequence in many types of host cell and those which direct expression of the nucleotide sequence only in certain host cells (*e.g.,* tissue-specific regulatory sequences). It will be appreciated by those skilled in the art that the design of the expression vector can depend on such factors as the choice of the host cell to be transformed, the level of expression of protein desired, and the like. The expression vectors of the invention can be introduced into host cells to thereby produce proteins or peptides, including fusion proteins or peptides, encoded by nucleic acids as described herein.

The recombinant expression vectors can be designed for expression of a polypeptide, or functional fragment thereof, in prokaryotic (*e.g., E. coli*) or eukaryotic cells (*e.g.,* insect cells {using baculovirus expression vectors}, yeast cells or mammalian cells). Suitable host cells are discussed further in Goeddel, *supra,* and include, for example, *E. coli* cells, *Bacillus* cells, *Saccharomyces* cells, *Pochia* cells, NS0 cells, COS cells, Chinese hamster ovary (CHO) cells or myeloma cells. The RNA or DNA also may be modified, for example, by substituting bases to optimize for codon usage in a particular host or by covalently joining to the coding sequence of a heterologous polypeptide. Such an approach would be the basis for developing a subunit vaccine. Alternatively, the recombinant expression vector can be transcribed and translated *in vitro.*

Another aspect of the disclosure pertains to host cells into which a recombinant vector of the invention has been introduced. The terms "host cell" and "recombinant host cell" are used interchangeably herein. It is understood that such terms refer not only to the particular subject cell but to the progeny or potential progeny of such a cell. Because certain modifications may occur in succeeding generations due to either mutation or environmental influences, such progeny may not, in fact, be identical to the parent cell, but are still included within the scope of the term as used herein.

Vector DNA can be introduced into prokaryotic or eukaryotic cells *via* conventional transformation or transfection techniques. As used herein, the terms "transformation" and "transfection" are intended to refer to a variety of art-recognized techniques for introducing foreign nucleic acid into a host cell, including calcium phosphate or calcium chloride co-precipitation, DEAE-dextran-mediated transfection, lipofection, or electroporation. Suitable methods for transforming or transfecting host cells can be found in Sambrook, *et al.* (*supra*), and other laboratory manuals.

### III. Acellular Tissue Matrices

The term "acellular tissue matrix," as used herein, refers generally to any tissue matrix that is substantially free of cells and/or cellular components. Skin, parts of skin (*e.g.,* dermis), and other tissues such as blood vessels, heart valves, fascia, cartilage, adipose tissue, bone, and nerve connective tissue may be used to create acellular matrices within the scope of the present disclosure. Acellular tissue matrices can be tested or evaluated to determine if they are substantially free of cell and/or cellular components in a number of ways. For example, processed tissues can be inspected with light microscopy to determine if cells (live or dead) and/or cellular components remain. In addition, certain assays can be used to identify the presence of cells or cellular components. For example, DNA or other nucleic acid assays can be used to quantify remaining nuclear materials within the tissue matrices. Generally, the absence of remaining DNA or other nucleic acids will be indicative of complete decellularization (*i.e.,* removal of cells and/or cellular components). Finally, other methods which identify cell-specific components (*e.g.,* surface antigens) can be used to determine if the tissue matrices are acellular. Skin, parts of skin (*e.g.,* dermis), and other tissues such as blood vessels, heart valves, fascia, cartilage, bone, and nerve connective tissue may be used to create acellular matrices within the scope of the present disclosure.

In general, the steps involved in the production of an acellular tissue matrix include harvesting the tissue from a donor (*e.g.,* an animal source) and cell removal under conditions that preserve biological and structural function. In certain embodiments, the process includes chemical treatment to stabilize the tissue and avoid biochemical and structural degradation together with or before cell removal. In various embodiments, the stabilizing solution arrests and prevents osmotic, hypoxic, autolytic, and proteolytic degradation, protects against microbial contamination, and reduces mechanical damage that can occur with tissues that contain, for example, smooth muscle components (*e.g.,* blood vessels). The stabilizing solution may contain an appropriate buffer, one or more antioxidants, one or more oncotic agents, one or more antibiotics, one or more protease inhibitors, and/or one or more smooth muscle relaxants.

The tissue is then placed in a decellularization solution to remove viable cells (*e.g.,* epithelial cells, endothelial cells, smooth muscle cells, and fibroblasts) from the structural matrix without damaging the biological and structural integrity of the collagen matrix. The decellularization solution may contain an appropriate buffer, salt, an antibiotic, one or more detergents (*e.g.,* TRITON X-100™, sodium deoxycholate, polyoxyethylene (20) sorbitan mono-oleate), one or more agents to prevent cross-linking, one or more protease inhibitors, and/or one or more enzymes. In some embodiments, the decellularization solution comprises 1% TRITON X-100™ in RPMI media with Gentamicin and 25 mM EDTA (ethylenediaminetetraacetic acid). In some embodiments, the tissue is incubated in the decellularization solution overnight at 37° C. with gentle shaking at 90 rpm. In certain embodiments, additional detergents may be used to remove fat from the tissue sample. For example, in some embodiments, 2% sodium deoxycholate is added to the decellularization solution.

After the decellularization process, the tissue sample is washed thoroughly with saline. In some exemplary embodiments, the decellularized tissue is then treated overnight at room temperature with a deoxyribonuclease (DNase) solution. In some embodiments, the tissue sample is treated with a DNase solution prepared in DNase buffer (20 mM HEPES (4-(2-hydroxyethyl)-1-piperazineethanesulfonic acid), 20 mM CaCl₂ and 20 mM MgCl₂). Optionally, an antibiotic solution (*e.g.,* Gentamicin) may be added to the DNase solution. Any suitable buffer can be used as long as the buffer provides suitable DNase activity.

After washing the tissue thoroughly with saline to remove the DNase solution, the tissue sample is contacted with a *T. reesei* or a *C. cellulolyticum* alpha-galactosidase.

After treatment with an alpha-galactosidase, an assay may be performed to determine if the collagen-containing tissue matrix has been altered such that a human immune response is reduced. A number of suitable assays may be performed. For example, suitable assays can include monocyte activation assays, phagocytosis assays, and oxidative burst assays.

In some embodiments, the assay may be performed on a segment or portion of the processed tissue, and other portions of the tissue may be used in subsequent medical or surgical procedures. In other embodiments, the assay may be performed on one or more samples from a batch of multiple samples, and samples not subjected to the assay may be subsequently selected for use in treating a patient.

In some embodiments, after the acellular tissue matrix is formed, histocompatible, viable cells are seeded in the acellular tissue matrix to produce a graft that may be further remodeled by the host. In some embodiments, histocompatible viable cells may be added to the matrices by standard in vitro cell co-culturing techniques prior to transplantation, or by *in vivo* repopulation following transplantation. *In vivo* repopulation can be by the recipient's own cells migrating into the acellular tissue matrix or by infusing or injecting cells obtained from the recipient or histocompatible cells from another donor into the acellular tissue matrix in situ. Various cell types can be used, including embryonic stem cells, adult stem cells (*e.g.* mesenchymal stem cells), and/or neuronal cells. In various embodiments, the cells can be directly applied to the inner portion of the acellular tissue matrix just before or after implantation. In certain embodiments, the cells can be placed within the acellular tissue matrix to be implanted, and cultured prior to implantation.

The present invention is further illustrated by the following examples, which should not be construed as further limiting.

### EXAMPLE 1: Identification of Microbial Alpha-Galactosidases for Removal of Terminal Alpha-1,3-Linked Galactose Residues From Tissue

Publically available databases were surveyed to identify commercially available alpha-galactosidases and microbial organisms that produce alpha-galactosidases.

Enzymes were purchased or partially purified and tested for alpha-glactosidase activity on p-nitrophenyl alpha-galatctosidase (pNGP) substrate. Briefly, samples were diluted in either phosphate buffer pH 6.0 or 20 mM HEPES, 1.2 M NaCl, 20 mM CaCl2, 20 mM MgCl2, pH 7.4. pNGP was then added to a final concentration of 1 mM and the samples incubated at 37C. Enzyme activity was assessed by measuring resulting absorbance at 405 nm.

Those microbial enzymes having activity on pNGP were subsequently tested for their ability to reduce and/or eliminate α-gal epitopes on tissue. The enzymes and the source of the alpha-galactosidase tested are listed below.
*Aspergillus niger* (partially purified materials were purchased from MarCor and Specialty Enzyme; tissue samples were tested with concentrations up to 20,000 U/L)
   *Agl*A
   *Agl*B
   *Agl*C (purified recombinant *Agl*C was purchased from Megazyme; tested on tissue at concentrations up to 100,000 U/L)
   *Agl* Unknown
*Trichoderma reesei* (purified material from MPBio having activity on tissue at 400 U/L, and crude and partially purified (Q-HiTrap) from crude culture supernatants)
   *Agl*I
   *Agl*II
   *Agl*III
*Guar* (purified recombinant (*E. coli*) form purchased from Megazyme; tested on tissue at concentrations up to 100,000 U/L)
*Phaseus*
*Cellvibrio mixtus* (recombinant (*E. coli*) form purchased from Prozomix; tested on tissue at concentrations up to 20,000 U/L)
*Clostridium cellulyticum* (recombinant (*E. coli*) form purchased from Prozomix; active on tissue at 4,000 U/L)
*Saccharomyces cerevisiae* (recombinant form purchased from CUSABIO and a proprietary source; tested on tissue at concentrations up to 20,000 U/L, or 50 mg/ml)
*Bacillus subtilis* (recombinant (*E. coli*) form purchased from Genway Biologics)
*Xanthomonas manihotis* (recombinant (*E. coli*) form purchased from New England BioLabs; tested on tissue at concentrations up to 50,000 U/L)
*Escherechia coli* (recombinant form purchased from U.S. biological; tested on tissue at concentrations up to 13 mg/ml)

Porcine skin was collected from an abattoir and split to 1.3 mm by physically removing the epidermis and subcutaneous fat. The remaining dermal tissue was decontaminated with peracetic acid. Following de-contamination, the tissue was processed under aseptic conditions. The dermal tissue was decellularized for 24 hours with detergents to remove viable cells. Cellular debris and residual chemicals are removed by washing in PBS. The resulting porcine acellular dermal matrix (pADM) was stored at 4°C until use.

Enzyme preparations from those microbes identified as having activity on pNGP were tested on porcine dermis in a phosphate buffer, pH 6.0 or LTM DNase buffer, pH 7.4 (20 mM HEPES, 1.2 M NaCl, 20 mM CaCl2, 20 mM MgCl2) for up to 24 hours. Histological sections were prepared and stained with the IB4 lectin which specifically recognizes the α-gal epitope. Of the multitude of microbial enzymes annotated and/or tested as having alpha-galactosidase activity on pNGP, only two alpha-galactosidases from *T. reesei* or a *C. cellulolyticum* were effective at removing the α-gal epitopes from tissues. Specifically, the tissue sections treated with partially purified and commercially available alpha-galactosidase from *T. reesei* were as effective as the coffee bean alpha-galactosidase at reducing staining specific for the α-gal epitopes on tissue (see, *e.g.,* Figures 1B-1E). In addition, recombinant *C. cellulolyticum* alpha-galactosidase was also as effective as the coffee bean alpha-galactosidase at reducing staining specific for the α-gal epitopes on tissue but demonstrated greater activity in reduced pH buffers (see, *e.g.,* Figures 1G-1J).

## Claims

1. An *in vitro* method for preparing a non-human tissue matrix for transplantation, comprising:
contacting a collagen-containing tissue matrix with an isolated *Trichoderma reesei* or *Clostridium cellulyticum* alpha-galactosidase in an amount and for a time sufficient to remove an α-gal epitope from the tissue matrix, thereby preparing the tissue matrix for transplantation into a human patient.

2. The method of claim 1, wherein the alpha-galactosidase is an isolated *Trichoderma reesei* alpha-galactosidase, and comprises an amino acid sequence having at least 85% identity to the entire amino acid sequence set forth in SEQ ID NO: 2.

3. The method of claim 1, wherein the alpha-galactosidase is an isolated *Trichoderma reesei* alpha-galactosidase, and comprises an amino acid sequence having at least 85% identity to the entire amino acid sequence set forth in a sequence selected from the group consisting of SEQ ID NOs: 4 and 6.

4. The method of claim 1, wherein the alpha-galactosidase is an isolated *Trichoderma reesei* alpha-galactosidase, and is encoded by a nucleic acid molecule comprising a nucleotide sequence having at least 85% identity to the entire nucleotide sequence set forth in a sequence selected from the group consisting of SEQ ID NOs:1, 3, and 5.

5. The method of claim 1, wherein the alpha-galactosidase is an isolated *Trichoderma reesei* alpha-galactosidase, and is encoded by a nucleic acid molecule, said nucleic acid molecule encoding a protein comprising an amino acid sequence having at least 85% identity to the entire amino acid sequence set forth in a sequence selected from the group consisting of SEQ ID NOs: 2, 4, and 6.

6. The method of claim 1, wherein the alpha-galactosidase is an isolated *Clostridium cellulyticum* alpha-galactosidase, and comprises an amino acid sequence having at least 85% identity to the entire amino acid sequence set forth in SEQ ID NO: 8.

7. The method of claim 1, wherein the alpha-galactosidase is an isolated *Clostridium cellulyticum* alpha-galactosidase, and is encoded by a nucleic acid molecule comprising a nucleotide sequence having at least 85% identity to the entire nucleotide sequence set forth in SEQ ID NO:7.

8. The method of claim 1, wherein the alpha-galactosidase is an isolated *Clostridium cellulyticum* alpha-galactosidase, and is encoded by a nucleic acid molecule, said nucleic acid molecule encoding a protein comprising an amino acid sequence having at least 85% identity to the entire amino acid sequence set forth in SEQ ID NO:8.

9. The method of any one of claims 1-8, wherein the tissue matrix is an acellular tissue matrix.

10. The method of any one of claims 1-9, wherein the tissue matrix:
(a) comprises a dermal tissue matrix; or
(b) is obtained from a tissue selected from fascia, pericardial tissue, dura, umbilical cord tissue, placental tissue, cardiac valve tissue, ligament tissue, tendon tissue, arterial tissue, venous tissue, neural connective tissue, urinary bladder tissue, ureter tissue, and intestinal tissue.

11. The method of any one of claims 1-10, further comprising treating the tissue matrix to remove at least some of the cells and cellular components from the tissue matrix.

12. The method of claim 11, wherein the tissue matrix is treated to remove substantially all cells and cellular components prior to contacting the tissue matrix with the alpha-galactosidase.

13. The method of claim 11, wherein substantially all the cells and cellular components are removed from the tissue matrix.

14. The method of any one of claims 1-13, wherein the at least one collagen-containing tissue matrix includes two or more tissue matrices.

15. The method of any one of claims 1-14, further comprising packaging the tissue matrix; and/or, further comprising sterilizing the tissue matrix.

## Patentansprüche

1. In-vitro-Verfahren zur Herstellung einer nicht menschlichen Gewebematrix zur Transplantation, umfassend:
Bringen einer collagenhaltigen Gewebematrix in Kontakt mit einer isolierten Alpha-Galactosidase von Trichoderma reesei oder Clostridium cellulyticum in einer Menge und für eine Zeit, die zum Entfernen eines α-Gal-Epitops von der Gewebematrix ausreichend sind, wodurch die Gewebematrix zur Transplantation in einen menschlichen Patienten hergestellt wird.

2. Verfahren nach Anspruch 1, wobei die Alpha-Galactosidase eine isolierte Alpha-Galactosidase von Trichoderma reesei ist und eine Aminosäuresequenz mit mindestens 85 % Identität mit der gesamten Aminosäuresequenz umfasst, die in SEQ ID NO: 2 dargestellt ist.

3. Verfahren nach Anspruch 1, wobei die Alpha-Galactosidase eine isolierte Alpha-Galactosidase von Trichoderma reesei ist und eine Aminosäuresequenz mit mindestens 85 % Identität mit der gesamten Aminosäuresequenz umfasst, die in einer Sequenz dargestellt ist, die ausgewählt ist aus der Gruppe bestehend aus SEQ ID NO: 4 und 6.

4. Verfahren nach Anspruch 1, wobei die Alpha-Galactosidase eine isolierte Alpha-Galactosidase von Trichoderma reesei ist und durch ein Nukleinsäuremolekül codiert wird, das eine Nukleotidsequenz mit mindestens 85 % Identität mit der gesamten Nukleotidsequenz umfasst, die in einer Sequenz dargestellt ist, die ausgewählt ist aus der Gruppe bestehend aus SEQ ID NO: 1, 3 und 5.

5. Verfahren nach Anspruch 1, wobei die Alpha-Galactosidase eine isolierte Alpha-Galactosidase von Trichoderma reesei ist und durch ein Nukleinsäuremolekül codiert wird, wobei das Nukleinsäuremolekül ein Protein codiert, das eine Aminosäuresequenz mit mindestens 85 % Identität mit der gesamten Aminosäuresequenz umfasst, die in einer Sequenz dargestellt ist, die ausgewählt ist aus der Gruppe bestehend aus SEQ ID NO: 2, 4 und 6.

6. Verfahren nach Anspruch 1, wobei die Alpha-Galactosidase eine isolierte Alpha-Galactosidase von Clostridium cellulyticum ist und eine Aminosäuresequenz mit mindestens 85 % Identität mit der gesamten Aminosäuresequenz umfasst, die in SEQ ID NO: 8 dargestellt ist.

7. Verfahren nach Anspruch 1, wobei die Alpha-Galactosidase eine isolierte Alpha-Galactosidase von Clostridium cellulyticum ist und durch ein Nukleinsäuremolekül codiert wird, das eine Nukleotidsequenz mit mindestens 85 % Identität mit der gesamten Nukleotidsequenz umfasst, die in SEQ ID NO: 7 dargestellt ist.

8. Verfahren nach Anspruch 1, wobei die Alpha-Galactosidase eine isolierte Alpha-Galactosidase von Clostridium cellulyticum ist und durch ein Nukleinsäuremolekül codiert wird, wobei das Nukleinsäuremolekül ein Protein codiert, das eine Aminosäuresequenz mit mindestens 85 % Identität mit der gesamten Aminosäuresequenz umfasst, die in SEQ ID NO: 8 dargestellt ist.

9. Verfahren nach einem der Ansprüche 1-8, wobei die Gewebematrix eine azelluläre Gewebematrix ist.

10. Verfahren nach einem der Ansprüche 1-9, wobei die Gewebematrix:
(a) eine Hautgewebematrix umfasst; oder
(b) aus einem Gewebe gewonnen ist, das ausgewählt ist aus Faszie, Herzbeutelgewebe, Dura, Nabelschnurgewebe, Plazentagewebe, Herzkappengewebe, Bändergewebe, Sehnengewebe, Arteriengewebe, Venengewebe, neuralem Bindegewebe, Harnblasengewebe, Harnleitergewebe und Darmgewebe.

11. Verfahren nach einem der Ansprüche 1-10, ferner umfassend Behandeln der Gewebematrix, um zumindest einige der Zellen und zellulären Komponenten von der Gewebematrix zu entfernen.

12. Verfahren nach Anspruch 11, wobei die Gewebematrix behandelt wird, um im Wesentlichen alle Zellen und zellulären Komponenten zu entfernen, bevor die Gewebematrix mit der Alpha-Galactosidase in Kontakt gebracht wird.

13. Verfahren nach Anspruch 11, wobei im Wesentlichen alle Zellen und zellulären Komponenten von der Gewebematrix entfernt werden.

14. Verfahren nach einem der Ansprüche 1-13, wobei die mindestens eine collagenhaltige Gewebematrix zwei oder mehr Gewebematrizen einschließt.

15. Verfahren nach einem der Ansprüche 1-14, ferner umfassend Verpacken der Gewebematrix; und/oder ferner umfassend Sterilisieren der Gewebematrix.

## Revendications

1. Procédé *in vitro* pour préparer une matrice tissulaire non humaine pour une transplantation, comprenant :
mettre en contact une matrice tissulaire contenant du collagène avec une alpha-galactosidase de *Trichoderma reesei* ou de *Clostridium cellulyticum* isolée dans une quantité et pendant une durée suffisantes pour retirer un épitope α-gal à partir de la matrice tissulaire, permettant ainsi de préparer la matrice tissulaire pour une transplantation dans un patient humain.

2. Procédé selon la revendication 1, dans lequel l'alpha-galactosidase est une alpha-galactosidase de *Trichoderma reesei* isolée, et comprend une séquence d'acides aminés ayant au moins 85 % d'identité avec la séquence d'acides aminés entière présentée dans SEQ ID NO : 2.

3. Procédé selon la revendication 1, dans lequel l'alpha-galactosidase est une alpha-galactosidase de *Trichoderma reesei* isolée, et comprend une séquence d'acides aminés ayant au moins 85 % d'identité avec la séquence d'acides aminés entière présentée dans une séquence choisie dans le groupe consistant en SEQ ID NOs : 4 et 6.

4. Procédé selon la revendication 1, dans lequel l'alpha-galactosidase est une alpha-galactosidase de *Trichoderma reesei* isolée, et est codée par une molécule d'acide nucléique comprenant une séquence nucléotidique ayant au moins 85 % d'identité avec la séquence nucléotidique entière présentée dans une séquence choisie dans le groupe consistant en SEQ ID NOs : 1, 3 et 5.

5. Procédé selon la revendication 1, dans lequel l'alpha-galactosidase est une alpha-galactosidase de *Trichoderma reesei* isolée, et est codée par une molécule d'acide nucléique, ladite molécule d'acide nucléique codant pour une protéine comprenant une séquence d'acides aminés ayant au moins 85 % d'identité avec la séquence d'acides aminés entière présentée dans une séquence choisie dans le groupe consistant en SEQ ID NOs : 2, 4 et 6.

6. Procédé selon la revendication 1, dans lequel l'alpha-galactosidase est une alpha-galactosidase de *Clostridium cellulyticum* isolée, et comprend une séquence d'acides aminés ayant au moins 85 % d'identité avec la séquence d'acides aminés entière présentée dans SEQ ID NO : 8.

7. Procédé selon la revendication 1, dans lequel l'alpha-galactosidase est une alpha-galactosidase de *Clostridium cellulyticum* isolée, et est codée par une molécule d'acide nucléique comprenant une séquence nucléotidique ayant au moins 85 % d'identité avec la séquence nucléotidique entière présentée dans SEQ ID NO : 7.

8. Procédé selon la revendication 1, dans lequel l'alpha-galactosidase est une alpha-galactosidase de *Clostridium cellulyticum* isolée, et est codée par une molécule d'acide nucléique, ladite molécule d'acide nucléique codant pour une protéine comprenant une séquence d'acides aminés ayant au moins 85 % d'identité avec la séquence d'acides aminés entière présentée dans SEQ ID NO : 8.

9. Procédé selon l'une quelconque des revendications 1 à 8, dans lequel la matrice tissulaire est une matrice tissulaire acellulaire.

10. Procédé selon l'une quelconque des revendications 1 à 9, dans lequel la matrice tissulaire :
(a) comprend une matrice tissulaire dermique ; ou
(b) est obtenue à partir d'un tissu choisi parmi un fascia, le tissu péricardique, une dura, le tissu de cordon ombilical, le tissu placentaire, le tissu de valvule cardiaque, le tissu de ligament, le tissu de tendon, le tissu artériel, le tissu veineux, le tissu conjonctif neuronal, le tissu de vessie urinaire, le tissu d'uretère et le tissu intestinal.

11. Procédé selon l'une quelconque des revendications 1 à 10, comprenant en outre le traitement de la matrice tissulaire pour retirer au moins une partie des cellules et des composants cellulaires à partir de la matrice tissulaire.

12. Procédé selon la revendication 11, dans lequel la matrice tissulaire est traitée pour retirer sensiblement toutes les cellules et tous les composants cellulaires avant la mise en contact de la matrice tissulaire avec l'alpha-galactosidase.

13. Procédé selon la revendication 11, dans lequel sensiblement toutes les cellules et tous les composants cellulaires sont retirés de la matrice tissulaire.

14. Procédé selon l'une quelconque des revendications 1 à 13, dans lequel ladite au moins une matrice tissulaire contenant du collagène comprend au moins deux matrices tissulaires.

15. Procédé selon l'une quelconque des revendications 1 à 14, comprenant en outre le conditionnement de la matrice tissulaire ; et/ou, comprenant en outre la stérilisation de la matrice tissulaire.
